(19) Europäisches Patentamt
European Patent Office
Office européen des brevets

(11) EP 3 932 974 B1

(12) EUROPEAN PATENT SPECIFICATION

(45) Date of publication and mention of the grant of the patent:
**22.02.2023 Bulletin 2023/08**

(21) Application number: **21171102.3**

(22) Date of filing: **29.04.2021**

(51) International Patent Classification (IPC):
***A61K 8/81*** *(2006.01)* ***A61Q 1/02*** *(2006.01)*
***C08J 3/12*** *(2006.01)* ***C08J 7/04*** *(2006.01)*
***A61K 8/02*** *(2006.01)*

(52) Cooperative Patent Classification (CPC):
**C08J 3/126; A61K 8/0241; A61K 8/817; A61Q 1/02; C08J 7/042;** A61K 2800/594; A61K 2800/63; C08J 2300/16; C08J 2301/12; C08J 2301/14; C08J 2367/02; C08J 2367/04; C08J 2433/00; C08J 2439/02; C08J 2443/04;

(Cont.)

# (54) BIODEGRADABLE RESIN PARTICLE

BIOLOGISCH ABBAUBARE HARZTEILCHEN

PARTICULE DE RÉSINE BIODÉGRADABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2020 JP 2020113307**
**13.11.2020 JP 2020189310**

(43) Date of publication of application:
**05.01.2022 Bulletin 2022/01**

(73) Proprietor: **Fujifilm Business Innovation Corp.**
**Tokyo (JP)**

(72) Inventors:
- **OKI, Masahiro**
**Tokyo (JP)**
- **YAO, Kenji**
**Tokyo (JP)**
- **YOSHIKAWA, Hideaki**
**Tokyo (JP)**
- **IWANAGA, Takeshi**
**Tokyo (JP)**
- **YOSHIDA, Kazusei**
**Tokyo (JP)**
- **TAGUCHI, Tetsuya**
**Tokyo (JP)**

(74) Representative: **Haseltine Lake Kempner LLP**
**Bürkleinstrasse 10**
**80538 München (DE)**

(56) References cited:
**EP-A1- 1 857 489**
**WO-A1-2013/166385**
**WO-A1-2015/048515**

- **DATABASE WPI Week 200465 Thomson Scientific, London, GB; AN 2004-664737 XP002804051, & JP 2004 256579 A (MITSUBISHI PAPER MILLS LTD) 16 September 2004 (2004-09-16)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 932 974 B1

(52) Cooperative Patent Classification (CPC): (Cont.)
C08J 2467/00; C08J 2475/04; C08J 2479/02;
C08J 2491/06

## Description

## Background

### Technical Field

The present invention relates to a biodegradable resin particle.

### Related Art

Patent Literature 1 discloses "a cellulose material having improved biodegradability by coating with a water-soluble polymer".

Patent Literature 1 discloses polyethyleneimine as the water-soluble polymer.

Patent Literature 2 discloses "a makeup method that changes an appearance of a skin, changes a touch feeling of the skin, and/or protects the skin, the makeup method including a step of applying, onto the skin, a self-sustaining beauty sheet containing at least one biocompatible and/or biodegradable hydrophobic polymer layer, in which the self-sustaining beauty sheet has a thickness of 10 nm to 1000 nm, preferably 30 nm to 500 nm, and more preferably 50 nm to 300 nm".

Patent Literature 2 discloses a layer containing polyethyleneimine as the hydrophobic polymer layer.

Patent Literature 3 discloses "a biodegradable resin composition containing: (A) a thermoplastic synthetic resin; (B) an inorganic filler; and (C) a biodegradable organic matter, in which a blending amount of the component (A) is 49 mass% or less based on the total amount of the composition, and a blending ratio ((B)/(C)) of the component (B) to the component (C) is in a range of 3/7 to 7/3 in terms of mass ratio".

Patent Literature 3 discloses that the biodegradable resin composition is blended with at least one selected from liquid paraffin, metal soap, silicone, a side-chain crystalline polyolefin, stearic acid, and polyglutamic acid as a binder component (D).

Patent Literature 4 discloses "compositions, and methods that aid particle transport in mucus".

Patent Literature 1: JP-A-2004-256579
Patent Literature 2: JP-T-2015-512863
Patent Literature 3: JP-A-2011-225643
Patent Literature 4: WO 2013/166385 A1

## Summary

An object of the present invention is to provide a biodegradable resin particle having a slow initial biodegradation rate while having a biodegradation rate over time, as compared with a biodegradable resin particle having only a layer containing at least one cationic resin of a polyalkyleneimine, a polyallylamine and a polyvinylamine on a surface of a base particle containing a biodegradable resin.

The present invention is defined in and by the appended claims.

Specific means for solving the above problems include the following aspects.

According to a first aspect of the present invention, there is provided a biodegradable resin particle, containing:

a base particle containing a biodegradable resin which is a resin having an aerobic condition biodegradation rate of 50% or more after one month as measured by a method according to ISO-14855-2 (2018);
a first layer on a surface of the base particle, the first layer containing at least one cationic resin of a polyalkyleneimine, a polyallylamine and a polyvinylamine; and
a second layer on the first layer, the second layer containing an anionic or nonionic hydrophobic compound wherein hydrophobic compound refers to a compound that imparts a water contact angle of a pellet in which the biodegradable resin particle is pelletized of 70° or more and 120° or less.

The anionic or nonionic hydrophobic compound may be at least one selected from the group consisting of a silicone compound, a hydrocarbon compound, a fatty acid compound, an acrylic resin, a polyester resin, and a urethane resin.

The silicone compound may be at least one selected from the group consisting of a dimethylpolysiloxane, a methylpolysiloxane, an MQ resin, and a silicone rubber.

The hydrocarbon compound may be at least one selected from the group consisting of a paraffin wax, a microcrystalline wax, a polyethylene wax, and a polypropylene wax.

The fatty acid compound may be at least one selected from the group consisting of a carnauba wax, a rice wax, a candelilla wax, a palm wax, a castor oil wax, a soybean oil wax, and a sunflower oil wax.

The polyalkyleneimine may be a polyalkyleneimine having a constituent unit having an alkylene group having 1 or more and 4 or less carbon atoms.

The polyalkyleneimine may have a constituent unit having an alkylene group having 1 or more and 4 or less carbon atoms and be a polyethyleneimine.

The biodegradable resin may be at least one selected from the group consisting of a cellulose resin and a polyester resin.

Accordingly, it is possible to provide a biodegradable resin particle having a biodegradation rate over time and a slow initial biodegradation rate, as compared with a biodegradable resin particle having only a layer containing at least one cationic resin of a polyalkyleneimine, a polyallylamine and a polyvinylamine on a surface of a base particle containing a biodegradable resin.

The biodegradable resin may be the cellulose resin.

Accordingly, it is possible to provide a biodegradable resin particle having a fast biodegradation rate over time and a slow initial biodegradation rate, as compared with a case where the biodegradable resin is a polyester resin.

The cellulose resin may be a cellulose acylate having two or more kinds of acyl groups.

Accordingly, it is possible to provide a biodegradable resin particle having a fast biodegradation rate over time and a slow initial biodegradation rate, as compared with a case where the cellulose resin is a cellulose acylate having one kind of acyl group.

The base particle may further contain at least one of a fatty acid ester and an aromatic compound, and the aromatic compound does not have a functional group that reacts with the cellulose resin and the aromatic compound has a long-chain aliphatic group and at least one of a phenolic hydroxy group and a monoglycidyl ether group that is directly bonded to an aromatic group of the aromatic compound.

Accordingly, it is possible to provide a biodegradable resin particle having biodegradability and excellent in hydrolysis resistance even when a cellulose resin and at least one of an aromatic compound and a fatty acid ester are contained as the biodegradable resin, as compared with a biodegradable resin particle having only a layer containing at least one cationic resin of a polyalkyleneimine, a polyallylamine and a polyvinylamine on a surface of a base particle containing a biodegradable resin.

The aromatic compound may be a cardanol compound.

The cardanol compound may be at least one compound selected from the group consisting of a compound represented by the following general formula (CDN1) and a compound represented by the following general formula (CDN1-e),

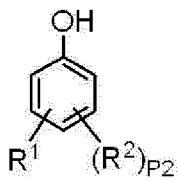 general formula (CDN1)

in which, in the general formula (CDN1), $R^1$ represents an alkyl group which may have a substituent or an unsaturated aliphatic group which has a double bond and may have a substituent, $R^2$ represents a hydroxy group, a carboxy group, an alkyl group which may have a substituent, or an unsaturated aliphatic group which has a double bond and may have a substituent, P2 represents an integer of 0 or more and 4 or less, and a plurality of $R^2{}_S$ may be the same group or different groups when P2 is 2 or more,

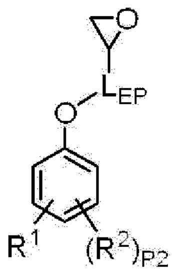 general formula (CDN1-e)

in which, in the general formula (CDN1-e), $L^{EP}$ represents a single bond or a divalent linking group, $R^1$ represents an

alkyl group which may have a substituent or an unsaturated aliphatic group which has a double bond and may have a substituent, $R^2$ represents a hydroxy group, a carboxy group, an alkyl group which may have a substituent, or an unsaturated aliphatic group which has a double bond and may have a substituent, P2 represents an integer of 0 or more and 4 or less, and a plurality of $R^2{}_S$ may be the same group or different groups when P2 is 2 or more.

Accordingly, it is possible to provide a biodegradable resin particle having biodegradability and excellent in hydrolysis resistance, as compared with a case where an alkyl-modified product of a phenol novolac type epoxy resin is applied as an aromatic compound.

The cellulose resin may be a cellulose acetate propionate and the aromatic compound may be a cardanol compound.

The cellulose resin may be a cellulose acetate butyrate and the base particle may contain the fatty acid ester.

Accordingly, it is possible to provide a biodegradable resin particle having biodegradability and excellent in hydrolysis resistance, as compared with a case where the base particle contains cellulose acetate butyrate as a cellulose resin and a cardanol compound as an aromatic compound, or a case where the base particle contains cellulose acetate propionate as a cellulose resin and a fatty acid ester.

A mass ratio of a content of the cationic resin in the first layer to a content of the anionic or nonionic hydrophobic compound in the second layer may be 0.1 or more and 10 or less.

Accordingly, it is possible to provide a biodegradable resin particle having a fast biodegradation rate over time and a slow initial biodegradation rate, as compared with a case where the mass ratio of the cationic resin to the hydrophobic compound is less than 0.1 or more than 10.

A content of the cationic resin with respect to the base particle may be 0.1 mass% or more and 10 mass% or less.

Accordingly, it is possible to provide a biodegradable resin particle having a fast biodegradation rate over time and a slow initial biodegradation rate, as compared with a case where a content of the cationic resin with respect to the base particle is less than 0.1 mass% or more than 10 mass%.

A number average molecular weight of the cationic resin may be 10,000 or more and 85,000 or less.

Accordingly, it is possible to provide a biodegradable resin particle having a fast biodegradation rate over time and a slow initial biodegradation rate, as compared with a case where the number average molecular weight of the cationic resin is less than 10,000 or more than 85,000.

## Detailed Description

Hereinafter, an exemplary embodiment as an example of the present invention will be described. These descriptions and Examples illustrate the exemplary embodiment, and do not limit the scope of the exemplary embodiment.

In the numerical ranges described in stages in this description, an upper limit or a lower limit described in one numerical range may be replaced with an upper limit or a lower limit of the numerical range described in other stages. Further, in the numerical ranges described in the present description, the upper limit or the lower limit of the numerical range may be replaced with values shown in Examples.

In the present description, the term "step" indicates not only an independent step, and even when a step cannot be clearly distinguished from other steps, this step is included in the term "step" as long as the intended purpose of the step is achieved.

Each component may contain a plurality of corresponding substances.

In a case of referring to the amount of each component, when there are a plurality of substances corresponding to each component, unless otherwise specified, it refers to the total amount of the plurality of substances.

The term "(meth)acryl" means at least one of acryl and methacryl, and the term "(meth)acrylate" means at least one of acrylate and methacrylate.

<Biodegradable Resin Particle>

The second, third and fourth Exemplary Embodiments are not claimed.

-First Exemplary Embodiment-

A biodegradable resin particle according to the first exemplary embodiment contains: a base particle containing a biodegradable resin; a first layer containing at least one cationic resin of a polyalkyleneimine, a polyallylamine and a polyvinylamine, which is present on a surface of the base particle; and a second layer containing an anionic or nonionic hydrophobic compound on the first layer.

With the above configuration, the biodegradable resin particle according to the first exemplary embodiment is a biodegradable resin particle having a biodegradation rate over time (for example, 12 months under aerobic conditions) and a slow initial biodegradation rate (for example, 3 months under aerobic conditions). The reasons are presumed as

follows.

The biodegradable resin particle is required to have a high biodegradation rate. However, when the biodegradation rate is excessively high, the durability of the resin particle decreases rapidly. That is, it is required to maintain the function as the resin particle for a certain period (for example, a period of several years during the period of use).

Therefore, the second layer containing an anionic or nonionic hydrophobic compound is provided on the surface of the base particle containing a biodegradable resin via the first layer containing at least one cationic resin of a polyalkyleneimine, a polyallylamine and a polyvinyl amine.

Under the presence of the first layer containing a cationic resin, an anionic or nonionic hydrophobic compound is adsorbed on the surface of the base particle to form a hydrophobic second layer. Therefore, the hydrophobicity of the surface of the biodegradable resin particle is increased. As a result, the initial biodegradation rate is slowed down, and the function as a resin particle is maintained for a certain period. Then, since the biodegradation of the hydrophobic compound in the second layer progresses with the passage of time, the biodegradability of the base particle containing a biodegradable resin is exhibited over time.

Therefore, it is presumed that the biodegradable resin particle according to the first exemplary embodiment is a biodegradable resin particle having a biodegradation rate over time and a slow initial biodegradation rate.

-Second Exemplary Embodiment-

A biodegradable resin particle according to the second exemplary embodiment contains: a base particle containing a biodegradable resin; and a compound layer on a surface of the base particle, in which a water contact angle of a pellet in which the biodegradable resin particle is pelletized is 70° or more and 120° or less.

With the above configuration, the biodegradable resin particle according to the second exemplary embodiment is a biodegradable resin particle having a biodegradation rate over time (for example, 12 months under aerobic conditions) and a slow initial biodegradation rate (for example, 3 months under aerobic conditions).

The reasons are that in the biodegradable resin particle, the surface of the compound layer exhibits hydrophobicity within the above contact angle range, the initial biodegradation rate is slowed down when the hydrolyzability of the biodegradable resin of the base particle is prevented, and the function as a resin particle is maintained for a certain period. Then, after a certain period, components of the compound layer of the biodegradable resin particle are decomposed, so that the biodegradability of the base particle containing a biodegradable resin is exhibited over time.

-Third Exemplary Embodiment-

A biodegradable resin particle according to the third exemplary embodiment contains: a base particle containing a biodegradable resin; and a compound layer on a surface of the base particle, in which an aerobic condition biodegradation rate after 3 months as measured by a method according to ISO-14855-2 (2018) is 20% or less.

With the above configuration, the biodegradable resin particle according to the third exemplary embodiment is a biodegradable resin particle having a biodegradation rate over time (for example, 12 months under aerobic conditions) and a slow initial biodegradation rate (for example, 3 months under aerobic conditions).

The reasons are that in the biodegradable resin particle, the compound layer reduces the aerobic condition biodegradation rate after the above 3 months, the initial biodegradation rate is slowed down when the hydrolyzability of the biodegradable resin of the base particle is prevented, and the function as a resin particle is maintained for a certain period. Then, after a certain period, components of the compound layer of the biodegradable resin particle are decomposed, so that the biodegradability of the base particle containing a biodegradable resin is exhibited over time.

-Fourth Exemplary Embodiment-

A biodegradable resin particle according to the fourth exemplary embodiment contains: a base particle containing a biodegradable resin; and a compound layer on a surface of the base particle, in which when a surface of the biodegradable resin particle is measured by X-ray photoelectron spectroscopy (XPS), a relationship between a carbon atomic weight Cs, a silicon atomic weight Sis, and an oxygen atomic weight Os satisfies an expression A:

$$(Cs + Sis)/Os \geq 3.$$

With the above configuration, the biodegradable resin particle according to the fourth exemplary embodiment is a biodegradable resin particle having a biodegradation rate over time (for example, 12 months under aerobic conditions) and a slow initial biodegradation rate (for example, 3 months under aerobic conditions).

The reasons are that in the biodegradable resin particle, the hydrophobicity of the surface of the biodegradable

resin particle is increased when the amounts of carbon atoms and silicon atoms present on the surface of the biodegradable resin particle are increased by the compound layer to satisfy the expression A, the initial biodegradation rate is slowed down when the hydrolyzability of the biodegradable resin of the base particle is prevented, and the function as a resin particle is maintained for a certain period. Then, after a certain period, components of the compound layer of the biodegradable resin particle are decomposed, so that the biodegradability of the base particle containing a biodegradable resin is exhibited over time.

Further, when the amounts of carbon atoms and silicon atoms present on the particle surface are increased, the oil lipophilicity of the surface of the biodegradable resin particle is improved, and the oil absorption rate of the resin particle is increased.

The biodegradable resin particles in the related art are excellent in degradability in the natural environment, but have a relatively hydrophilic structure, and therefore have low compatibility with oil or have a low oil absorbency. For example, when the biodegradable resin particles in the related art are added to cosmetics or the like, if the oil absorbency is low, the smeared makeup is likely to occur due to sebum or the like. Further, when the biodegradable resin particles in the related art are added to paints, if the oil absorbency is low and the compatibility with the oil is poor, a phenomenon of agglutination and solidification occurs. Therefore, the biodegradable resin particles having a high oil absorption rate are useful in the fields of cosmetics, paints, etc., because of being less likely to smear the makeup even when added to cosmetics and are less likely to aggregate even when added to paints.

Here, examples of the compound layer in the biodegradable resin particles according to the second to fourth exemplary embodiments include a two-layer compound layer including a first layer containing at least one cationic resin of a polyalkyleneimine, a polyallylamine and a polyvinyl amine and a second layer containing an anionic or nonionic hydrophobic compound on the first layer.

However, the compound layer in the biodegradable resin particles according to the second and third exemplary embodiments is not particularly limited as long as the compound layer is a compound layer to which the above characteristics of the biodegradable resin particles according to the second and third exemplary embodiments are imparted.

Hereinafter, the biodegradable resin particle corresponding to any of the biodegradable resin particles according to the first to fourth exemplary embodiments (hereinafter, also referred to as "biodegradable resin particle according to the present exemplary embodiment") will be described in detail. However, an example of the biodegradable resin particle of the present invention may be a biodegradable resin particle corresponding to any of the biodegradable resin particles according to the first to fourth exemplary embodiments.

Hereinafter, the biodegradable resin particle according to the present exemplary embodiment will be described in detail.

[Base Particle]

The base particle is a particle for which the first layer and the second layer are formed, and contain a biodegradable resin.

Examples of the base particle include a particle containing a biodegradable resin as a main component. Specific examples thereof include a particle containing preferably 90 mass%, more preferably 95 mass%, still more preferably 98 mass%, and particularly preferably 100 mass% of a biodegradable resin with respect to the entire base particle.

-Biodegradable Resin-

The biodegradable resin is a resin that is decomposed into water and carbon dioxide by microorganisms. Specifically, the biodegradable resin means a resin having an aerobic condition biodegradation rate of 50% or more after one month as measured by a method according to ISO-14855-2 (2018).

Examples of the biodegradable resin include a polyester resin, a natural polymer, and a polyvinyl alcohol.

Examples of the polyester resin include an aliphatic polyester resin and an aliphatic aromatic polyester resin.

Examples of the aliphatic polyester resin include polylactic acid (PLA), polyglycolic acid (PGA), and polyhydroxyalkanoic acids such as polyhydroxybutyrate, poly(3-hydroxybutyrate-co-3-hydroxyhexanoate) (PHBH), polycaprolactone, polybutylene succinate (PBS), polybutylene succinate/adipate (PBSA), and polyethylene succinate (PBA).

Examples of the aliphatic aromatic polyester resin include a polybutylene adipate/terephthalate copolymer resin (PBAH), and a polytetramethylene adipate/terephthalate copolymer resin.

Examples of the natural polymer include starch, cellulose, chitin, chitosan, gluten, gelatin, zein, soy protein, collagen, and keratin.

Among these, the biodegradable resin is preferably at least one selected from the group consisting of a cellulose resin and a polyester resin, and more preferably a cellulose resin, from the viewpoint of improving the biodegradation rate over time.

The cellulose resin is preferably cellulose acylate. The cellulose acylate is a cellulose derivative in which at

least a part of hydroxy groups in cellulose is substituted (in other words, acylated) by an acyl group. The acyl group is a group having a structure of $-COR^{AC}$ ($R^{AC}$ represents a hydrogen atom or a hydrocarbon group).

The cellulose acylate is, for example, a cellulose derivative represented by the following general formula (CA).

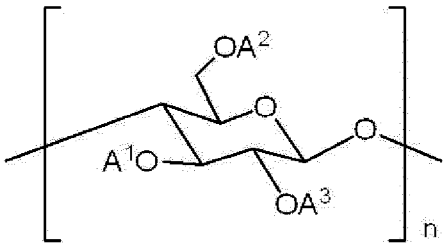
general formula (CA)

In the general formula (CA), $A^1$, $A^2$ and $A^3$ independently represent a hydrogen atom or an acyl group, and n represents an integer of 2 or more. However, at least a part of n $A^1$s, n $A^2$s and n $A^3$s represents an acyl group. The n $A^1{}_S$ in the molecule may be all the same as, partially the same as, or different from each other. Similarly, the n $A\ ^2{}_S$ may be all the same as, partially the same as, or different from each other, and the n $A^3$s in the molecule may be all the same as, partially the same as, or different from each other.

As for the acyl group represented by $A^1$, $A^2$ and $A^3$, the hydrocarbon group in the acyl group may be linear, branched or cyclic, and is preferably linear or branched, and more preferably linear.

As the acyl group represented by $A^1$, $A^2$ and $A^3$, the hydrocarbon group in the acyl group may be a saturated hydrocarbon group or an unsaturated hydrocarbon group, and is more preferably a saturated hydrocarbon group.

The acyl group represented by $A^1$, $A^2$ and $A^3$ is preferably an acyl group having 1 or more and 6 or less carbon atoms. That is, the cellulose acylate is preferably a cellulose acylate having an acyl group having 1 or more and 6 or less carbon atoms.

The acyl group represented by $A^1$, $A^2$ and $A^3$ may be a group in which a hydrogen atom in the acyl group is substituted with a halogen atom (for example, a fluorine atom, a bromine atom, and an iodine atom), an oxygen atom, a nitrogen atom or the like, but is preferably unsubstituted.

Examples of the acyl group represented by $A^1$, $A^2$ and $A^3$ include a formyl group, an acetyl group, a propionyl group, a butyryl group (butanoyl group), a propenoyl group, and a hexanoyl group. Among these, the acyl group is more preferably an acyl group having 2 or more and 4 or less carbon atoms, and still more preferably an acyl group having 2 or 3 carbon atoms, from the viewpoint of improving the biodegradation rate of the resin particle.

Examples of the cellulose acylate include cellulose acetate (such as cellulose monoacetate, cellulose diacetate (DAC), and cellulose triacetate), cellulose acetate propionate (CAP), and cellulose acetate butyrate (CAB).

The cellulose acylate is preferably a cellulose acylate having two or more kinds of acyl groups from the viewpoint of improving the biodegradation rate of the resin particle. Specifically, the cellulose acylate is preferably cellulose acetate propionate (CAP) and cellulose acetate butyrate (CAB), and more preferably cellulose acetate propionate (CAP) from the viewpoint of improving the biodegradation rate of the resin particle.

The cellulose acylate may be used alone or in combination of two or more thereof.

The weight-average degree of polymerization of the cellulose acylate is preferably 200 or more and 1000 or less, more preferably 500 or more and 1000 or less, and still more preferably 600 or more and 1000 or less.

The weight-average degree of polymerization of the cellulose acylate is determined from the weight average molecular weight (Mw) by the following procedure.

First, the weight average molecular weight (Mw) of the cellulose acylate is measured by gel permeation chromatography (GPC apparatus: HLC-8320GPC manufactured by Tosoh Corporation, column: TSKgel$\alpha$-M) in terms of polystyrene using tetrahydrofuran.

Next, the weight average molecular weight (Mw) of the cellulose acylate is divided by a molecular weight of a constituent unit of the cellulose acylate to determine the degree of polymerization of the cellulose acylate. For example, in a case where the substituent of the cellulose acylate is an acetyl group, the molecular weight of the constituent unit is 263 when the degree of substitution is 2.4 and 284 when the degree of substitution is 2.9.

From the viewpoint of improving the biodegradation rate of the resin particle, the degree of substitution of the cellulose acylate is preferably 2.1 or more and 2.9 or less, more preferably 2.2 or more and 2.9 or less, still more preferably 2.3 or more and 2.9 or less, and particularly preferably 2.6 or more and 2.9 or less.

In the cellulose acetate propionate (CAP), the ratio of the degree of substitution of the acetyl group to the propionyl group (acetyl group/propionyl group) is preferably 0.01 or more and 1 or less, and more preferably 0.05 or more and 0.1 or less, from the viewpoint of improving the biodegradation rate of the resin particle.

In the cellulose acetate butyrate (CAB), the ratio of the degree of substitution of the acetyl group to the butyryl

group (acetyl group/butyryl group) is preferably 0.05 or more and 3.5 or less, and more preferably 0.5 or more and 3.0 or less, from the viewpoint of improving the biodegradation rate of the resin particle.

The degree of substitution of the cellulose acylate is an index indicating a degree to which the hydroxy group of cellulose is substituted with an acyl group. That is, the degree of substitution is an index indicating a degree of acylation of the cellulose acylate. Specifically, the degree of substitution means an intramolecular average of the number of substitution in which three hydroxy groups in a D-glucopyranose unit of the cellulose acylate are substituted with acyl group. The degree of substitution is determined from a ratio of a peak integral of a cellulose-derived hydrogen to peak integral of an acyl group-derived hydrogen with $^1$H-NMR (JMN-ECA manufactured by JEOL RESONANCE Co., Ltd.).

The biodegradable resin may be used alone or in combination of two or more thereof.

-Other Components-

The base particle may contain other components.

Examples of other components include plasticizers, flame retardants, compatibilizers, mold release agents, light-resistant agents, weatherproofing agents, colorants, pigments, modifiers, drip inhibitors, antistatic agents, hydrolysis inhibitors, fillers, reinforcing agents (glass fiber, carbon fiber, talc, clay, mica, glass flakes, milled glass, glass beads, crystalline silica, alumina, silicon nitride, aluminum nitride, boron nitride, etc.), acid acceptors for preventing acid release (oxides such as magnesium oxide and aluminum oxide; metal hydroxides such as magnesium hydroxide, calcium hydroxide, aluminum hydroxide and hydrotalcite; calcium carbonate; talcite; etc.), and reactive trapping agents (epoxy compounds, acid anhydride compounds, carbodiimide, etc.).

The content of the other components is preferably 0 mass% or more and 5 mass% or less with respect to the total amount of the base particle. Here, "0 mass%" means that the base particle does not contain other components.

Examples of the plasticizer include an ester compound, camphor, a metal soap, a polyol, and a polyalkylene oxide. The plasticizer is preferably an ester compound from the viewpoint of improving the mechanical properties of the resin particle. The plasticizer may be used alone or in combination of two or more thereof.

Examples of the ester compound include fatty acid esters (e.g., adipate ester, citrate ester, sebacate ester, azelate ester, phthalate ester, and acetate ester), phosphate esters, condensed phosphate esters, glycol esters (e.g., benzoate glycol ester), and modified fatty acid esters (e.g., epoxidized fatty acid ester). Examples of the above ester include monoesters, diesters, triesters, and polyesters. Among these, dicarboxylic acid diesters (adipic acid diester, sebacic acid diester, azelaic acid diester, phthalic acid diester, etc.) are preferred.

The plasticizer is preferably adipate ester. The adipate ester has a high affinity for cellulose acylate, and by being dispersed in a state close to uniform with cellulose acylate, has thermal fluidity further improved as compared with other plasticizers.

As the adipate ester, a mixture of the adipate ester and other components may be used. Examples of commercially available products of the mixture include Daifatty 101 manufactured by DAIHACHI CHEMICAL INDUSTRY CO., LTD.

Examples of the fatty acid esters such as citrate ester, sebacate ester, azelate ester, phthalate ester, and acetate ester include esters of fatty acids and alcohols. Examples of the alcohol include: monohydric alcohols such as methanol, ethanol, propanol, butanol, and 2-ethylhexanol; and polyhydric alcohols such as glycerin, polyglycerin (diglycerin, etc.), pentaerythritol, ethylene glycol, diethylene glycol, propylene glycol, butylene glycol, trimethylolpropane, trimethylolethane, and sugar alcohol.

Examples of the glycol in the benzoate glycol ester include ethylene glycol, diethylene glycol, and propylene glycol.

The epoxidized fatty acid ester is an ester compound having a structure in which the carbon-carbon unsaturated bond of the unsaturated fatty acid ester is epoxidized (that is, oxacyclopropane). Examples of the epoxidized fatty acid ester include esters of fatty acids and alcohols in which some or all of the carbon-carbon unsaturated bond in unsaturated fatty acids (e.g., oleic acid, palmitoleic acid, vaccenic acid, linoleic acid, linolenic acid, and nervonic acid) are epoxidized. Examples of the alcohol include: monohydric alcohols such as methanol, ethanol, propanol, butanol, and 2-ethylhexanol; and polyhydric alcohols such as glycerin, polyglycerin (diglycerin, etc.), pentaerythritol, ethylene glycol, diethylene glycol, propylene glycol, butylene glycol, trimethylolpropane, trimethylolethane, and sugar alcohol.

The ester compound as a plasticizer has a molecular weight (or weight average molecular weight) of preferably 200 or more and 2000 or less, more preferably 250 or more and 1500 or less, and still more preferably 280 or more and 1000 or less. Unless otherwise specified, the weight average molecular weight of the ester compound is a value measured according to the method for measuring the weight average molecular weight of the cellulose acylate.

Here, when a cellulose resin is applied as the biodegradable resin, it is particularly preferably that the base particle contains the cellulose resin and, as a plasticizer, at least one of a fatty acid ester (hereinafter, also referred to as "fatty acid ester (B2)") and an aromatic compound (hereinafter, also referred to as "aromatic compound (B1)") that does not have a functional group that reacts with the cellulose resin and has a long-chain aliphatic group and at least

one of a phenolic hydroxy group and a monoglycidyl ether group that is directly bonded to an aromatic group of the aromatic compound.

The cellulose resin is less likely to hydrolyze by itself, and is difficult to mold because of being inferior in flexibility, so that it is preferable to add a plasticizer to the cellulose resin in order to impart flexibility. However, the cellulose resin to which the plasticizer is added is easily hydrolyzed. Therefore, coating the base particle containing the cellulose resin and the plasticizer with the second layer containing a hydrophobic compound is effective in imparting hydrolysis resistance while ensuring biodegradability.

On the other hand, in order to slow down the initial biodegradation rate, it is preferable that the base particle is firmly coated with the second layer containing a hydrophobic compound.

In this regard, when at least one of the aromatic compound (B1) and the fatty acid ester (B2) is used as the plasticizer to be contained in the base particle, the first layer containing a cationic resin, which functions as an adhesive layer between the surface of the base particle and the second layer containing a hydrophobic compound, is more firmly coated on the surface of the base particle. As a result, the base particle can be more firmly coated with the second layer containing a hydrophobic compound, and high hydrolysis resistance can be imparted to the biodegradable resin particle while ensuring biodegradability. The reasons are presumed as follows.

Since the aromatic compound (B1) has a benzene ring, the benzene ring attracts electrons and the OH group becomes acidic. Therefore, the reactivity with a cationic compound is high. Further, since the fatty acid ester (B2) is a compound that is acidic, it has high reactivity with a cationic compound. As a result, the first layer containing a cationic resin is more firmly coated on the surface of the base particle.

Therefore, it is presumed that the base particle can be more firmly coated with the second layer containing a hydrophobic compound, and high hydrolysis resistance can be imparted to the biodegradable resin particle while ensuring biodegradability. As a result, the biodegradable resin particle has excellent hydrolysis resistance while ensuring biodegradability.

In particular, when cellulose acetate propionate is applied as the cellulose resin, it is preferable to apply a cardanol compound as the aromatic compound used as the plasticizer from the viewpoint of improving the hydrolysis resistance while ensuring the biodegradability.

From the same viewpoint, when cellulose acetate propionate is applied as the cellulose resin, it is preferable to apply an aliphatic ester as the plasticizer.

Hereinafter, the aromatic compound (B1) will be described.

The aromatic compound (B1) is an aromatic compound that does not have a functional group that reacts with the cellulose resin and has a long-chain aliphatic group and at least one of a phenolic hydroxy group and a monoglycidyl ether group that is directly bonded to an aromatic group of the aromatic compound.

That is, the aromatic compound (B1) is a compound that does not have a functional group that reacts with the cellulose acylate and has a long-chain aliphatic group and at least one of a phenolic hydroxy group and a monoglycidyl ether group.

Here, examples of the long-chain aliphatic group include saturated aliphatic groups (that is, alkyl group) having 6 or more and 30 or less (preferably 10 or more and 20 or less) carbon atoms and unsaturated aliphatic groups (that is, alkenyl group and alkynyl group) having 6 or more and 30 or less (preferably 10 or more and 20 or less) carbon atoms. The aliphatic group may be linear, branched or cyclic, and is preferably linear or branched, and more preferably linear.

Examples of the aromatic compound (B1) include a compound in which a phenolic hydroxy group is substituted on a monocyclic ring, a condensed ring which is a polycyclic ring having two or more aromatic rings, a polynuclear ring which is a polycyclic ring in which aromatic rings are linked by a carbon-carbon bond, or a heterocyclic ring which is a monocyclic ring of a heterocyclic ring, a condensed ring containing a heterocyclic ring, a polynuclear ring containing a heterocyclic ring, etc. together with a long-chain aliphatic group.

Specific examples of the aromatic compound (B1) include a cardanol compound, a phenalkamine compound, a phenol resin, a phenol novolac type epoxy resin, a phenol resol type epoxy resin, a phenol-modified palm oil, a phenol-modified soybean oil, and a phenol-modified flaxseed oil.

Among these, a cardanol compound is preferred as the aromatic compound (B1) from the viewpoint of improving biodegradability.

The cardanol compound refers to a component contained in a naturally-derived compound made from cashew (for example, a compound represented by the following structural formulas (b-1) to (b-4)) or a derivative of the component.

(b-1)

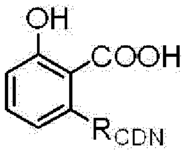

(b-2)

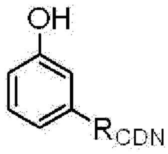

(b-3)

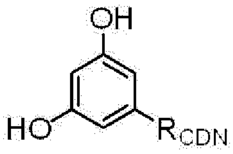

(b-4)

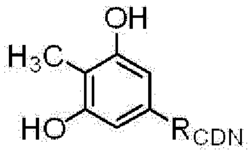

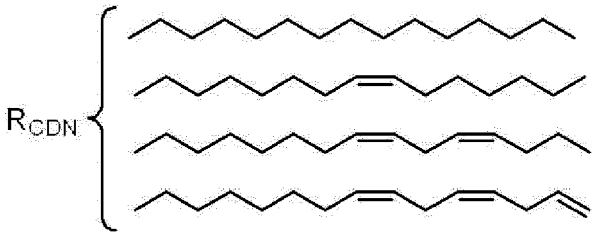

The cardanol compound may be a mixture of a naturally-derived compound made from cashew (hereinafter, also referred to as "cashew-derived mixture").

The cardanol compound may be a derivative of the cashew-derived mixture. Examples of the derivative of the cashew-derived mixture include the following mixtures and pure substances.

- A mixture prepared by adjusting the composition ratio of each component in the cashew-derived mixture
- A pure substance obtained by isolating only a specific component from the cashew-derived mixture
- A mixture containing a modified product obtained by modifying components in the cashew-derived mixture
- A mixture containing a polymer obtained by polymerizing components in the cashew-derived mixture
- A mixture containing a modified polymer obtained by modifying and polymerizing components in the cashew-derived mixture
- A mixture containing a modified product obtained by further modifying components in the mixture whose composition ratio is adjusted
- A mixture containing a polymer obtained by further polymerizing components in the mixture whose composition ratio is adjusted
- A mixture containing a modified polymer obtained by further modifying and polymerizing components in the mixture whose composition ratio is adjusted
- A modified product obtained by further modifying the isolated pure substance
- A polymer obtained by further polymerizing the isolated pure substance
- A modified polymer obtained by further modifying and polymerizing the isolated pure substance

Here, a pure substance includes a multimer such as a dimer and a trimer.

The cardanol compound is preferably at least one compound selected from the group consisting of a compound represented by a general formula (CDN1) and a polymer obtained by polymerizing the compound represented by the general formula (CDN1), from the viewpoint of improving the biodegradation rate of the resin particle.

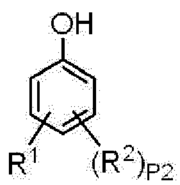 general formula (CDN1)

In the general formula (CDN1), $R^1$ represents an alkyl group which may have a substituent or an unsaturated aliphatic group which has a double bond and may have a substituent. $R^2$ represents a hydroxy group, a carboxy group, an alkyl group which may have a substituent, or an unsaturated aliphatic group which has a double bond and may have a substituent. P2 represents an integer of 0 or more and 4 or less. When P2 is 2 or more, a plurality of $R^2$s may be the same group or different groups.

In the general formula (CDN1), the alkyl group which may have a substituent and represented by $R^1$ is preferably an alkyl group having 3 or more and 30 or less carbon atoms, more preferably an alkyl group having 5 or more and 25

or less carbon atoms, and still more preferably an alkyl group having 8 or more and 20 or less carbon atoms.

Examples of the substituent include a hydroxy group; a substituent containing an ether bond such as an epoxy group and a methoxy group; and a substituent containing an ester bond such as an acetyl group and a propionyl group.

Examples of the alkyl group which may have a substituent include a pentadecane-1-yl group, a heptane-1-yl group, an octane-1-yl group, a nonane-1-yl group, a decane-1-yl group, an undecane-1-yl group, a dodecane-1-yl group, and a tetradecane-1-yl group.

In the general formula (CDN1), the unsaturated aliphatic group which has a double bond and may have a substituent and represented by $R^1$ is preferably an unsaturated aliphatic group having 3 or more and 30 or less carbon atoms, more preferably an unsaturated aliphatic group having 5 or more and 25 or less carbon atoms, and still more preferably an unsaturated aliphatic group having 8 or more and 20 or less carbon atoms.

The number of the double bond contained in the unsaturated aliphatic group is preferably 1 or more and 3 or less.

Examples of the substituent include those listed as the substituent of the above alkyl group.

Examples of the unsaturated aliphatic group which has a double bond and may have a substituent include a pentadeca-8-en-1-yl group, a pentadeca-8,11-diene-1-yl group, a pentadeca-8,11,14-trien-1-yl group, a pentadeca-7-en-l-yl group, a pentadeca-7,10-diene-1-yl group, and a pentadeca-7,10,14-trien-1-yl group.

In the general formula (CDN1), $R^1$ is preferably a pentadeca-8-ene-1-yl group, a pentadeca-8,11-diene-1-yl group, a pentadeca-8,11,14-triene-1-yl group, a pentadeca-7-ene-1-yl group, a pentadeca-7,10-diene-1-yl group, and a pentadeca-7,10,14-triene-1-yl group.

In the general formula (CDN1), similarly, preferred examples of the alkyl group which may have a substituent and the unsaturated aliphatic group which has a double bond and may have a substituent, which are represented by $R^2$, include those listed as the alkyl group which may have a substituent and the unsaturated aliphatic group which has a double bond and may have a substituent, which are represented by $R^1$.

The compound represented by the general formula (CDN1) may be further modified. For example, the compound may be epoxidized. Specifically, from the viewpoint of improving the biodegradation rate of the resin particle, the compound is preferably a compound having a structure in which the hydroxy group of the compound represented by the general formula (CDN1) is substituted with the following group (EP), i.e., a compound represented by the following general formula (CDN1-e).

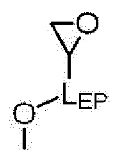

group (EP)

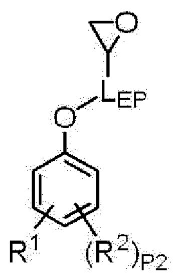

general formula (CDN1-e)

In the group (EP) and the general formula (CDN1-e), $L_{EP}$ represents a single bond or a divalent linking group. In the general formula (CDN1-e), $R^1$, $R^2$ and P2 have the same meaning with $R^1$, $R^2$ and P2 in the general formula (CDN1), respectively.

In the group (EP) and the general formula (CDN1-e), examples of the divalent linking group represented by $L_{EP}$ include an alkylene group which may have a substituent (preferably an alkylene group having 1 or more and 4 or less carbon atoms, and more preferably an alkylene group having 1 carbon atom), and a $-CH_2CH_2OCH_2CH_2-$ group.

Examples of the above substituent include those listed as the substituent of $R^1$ in the general formula (CDN1).

$L_{EP}$ is preferably a methylene group.

The polymer obtained by polymerizing the compound represented by the general formula (CDN1) refers to a polymer obtained by polymerizing at least two compounds represented by the general formula (CDN1) with or without a linking group.

Examples of the polymer obtained by polymerizing the compound represented by the general formula (CDN1) include a compound represented by the following general formula (CDN2).

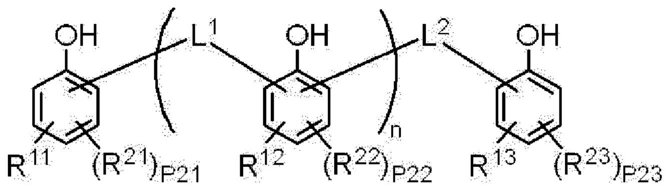 general formula (CDN2)

In the general formula (CDN2), $R^{11}$, $R^{12}$ and $R^{13}$ each independently represent an alkyl group which may have a substituent or an unsaturated aliphatic group which has a double bond and may have a substituent. $R^{21}$, $R^{22}$ and $R^{23}$ each independently represent a hydroxy group, a carboxy group, an alkyl group which may have a substituent, or an unsaturated aliphatic group which has a double bond and may have a substituent. P21 and P23 each independently represent an integer of 0 or more and 3 or less, and P22 represents an integer of 0 or more and 2 or less. $L^1$ and $L^2$ each independently represent a divalent linking group. n represents an integer of 0 or more and 10 or less. A plurality of $R^{21}$s when P21 is 2 or more may be the same group or different groups, a plurality of $R^{22}{}_S$ when P22 is 2 or more may be the same group or different groups, and a plurality of $R^{21}{}_S$ when P23 is 2 or more may be the same group or different groups. A plurality of $R^{12}{}_S$ when n is 2 or more may be the same group or different groups, a plurality of $R^{22}{}_S$ when n is 2 or more may be the same group or different groups, and a plurality of $L^1$s when n is 2 or more may be the same group or different groups, and a plurality of P22s when n is 2 or more may be the same number or different numbers.

In the general formula (CDN2), preferred examples of the alkyl group which may have a substituent and the unsaturated aliphatic group which has a double bond and may have a substituent, which are represented by $R^{11}$, $R^{12}$, $R^{13}$, $R^{21}$, $R^{22}$ and $R^{23}$, include those listed as $R^1$ in the general formula (CDN1).

In the general formula (CDN2), examples of the divalent linking group represented by $L^1$ and $L^2$ include an alkylene group which may have a substituent (preferably an alkylene group having 2 or more and 30 or less carbon atoms, and more preferably an alkylene group having 5 or more and 20 or less carbon atoms).

Examples of the above substituent include those listed as the substituent of $R^1$ in the general formula (CDN1).

In the general formula (CDN2), n is preferably 1 or more and 10 or less, and more preferably 1 or more and 5 or less.

The compound represented by the general formula (CDN2) may be further modified. For example, the compound may be epoxidized. Specifically, the compound may be a compound having a structure in which the hydroxy group of the compound represented by the general formula (CDN2) is substituted with the group (EP), i.e., a compound represented by the following general formula (CDN2-e).

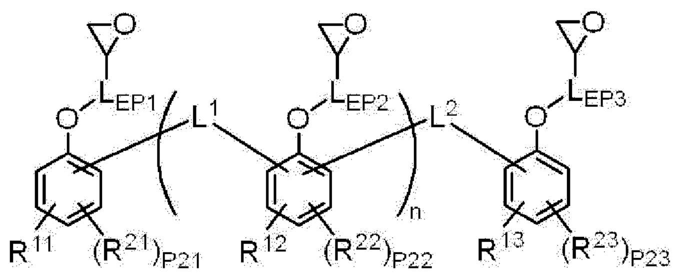 general formula (CDN2-e)

In the general formula (CDN2-e), $R^{11}$, $R^{12}$, $R^{13}$, $R^{21}$, $R^{22}$, $R^{23}$, P21, P22, P23, $L^1$, $L^2$ and n have the same meaning with $R^{11}$, $R^{12}$, $R^{13}$, $R^{21}$, $R^{22}$, $R^{23}$, P21, P22, P23, $L^1$, $L^2$ and n in the general formula (CDN2), respectively.

In the general formula (CDN2-e), $L_{EP1}$, $L_{EP2}$ and $L_{EP3}$ each independently represent a single bond or a divalent linking group. When n is 2 or more, a plurality of $L_{EP2}S$ may be the same group or different groups.

In the general formula (CDN2-e), preferred examples of the divalent linking group represented by $L_{EP1}$, $L_{EP2}$ and $L_{EP3}$ include those listed as the divalent linking group represented by $L_{EP}$ in the general formula (CDN1-e).

The polymer obtained by polymerizing the compound represented by the general formula (CDN1) may be, for example, a polymer obtained by three-dimensionally crosslinking and polymerizing at least three compounds represented by the general formula (CDN1) with or without a linking group. Examples of the polymer obtained by three-dimensionally crosslinking and polymerizing the compounds represented by the general formula (CDN1) include a compound represented by the following structural formula.

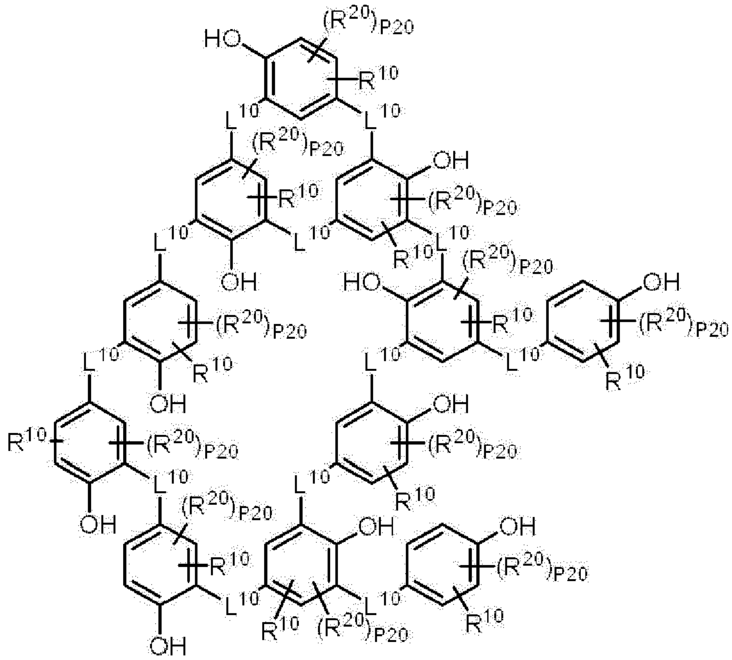

In the above structural formula, $R^{10}$, $R^{20}$ and P20 have the same meaning with $R^1$, $R^2$ and P2 in the general formula (CDN1), respectively. $L^{10}$ represents a single bond or a divalent linking group. A plurality of $R^{10}$s may be the same group or different groups, a plurality of $R^{20}{}_S$ may be the same group or different groups, and a plurality of $L^{10}$s may be the same group or different groups. A plurality of P20s may be the same number or different numbers.

In the above structural formula, examples of the divalent linking group represented by $L^{10}$ include an alkylene group which may have a substituent (preferably an alkylene group having 2 or more and 30 or less carbon atoms, and more preferably an alkylene group having 5 or more and 20 or less carbon atoms).

Examples of the above substituent include those listed as the substituent of $R^1$ in the general formula (CDN1).

The compound represented by the above structural formula may be further modified, for example, epoxidized. Specifically, the compound may be a compound having a structure in which the hydroxy group of the compound represented by the above structural formula is substituted with the group (EP), for example, a compound represented by the following structural formula, that is, a polymer obtained by three-dimensionally crosslinking and polymerizing the compound represented by the general formula (CDN1-e).

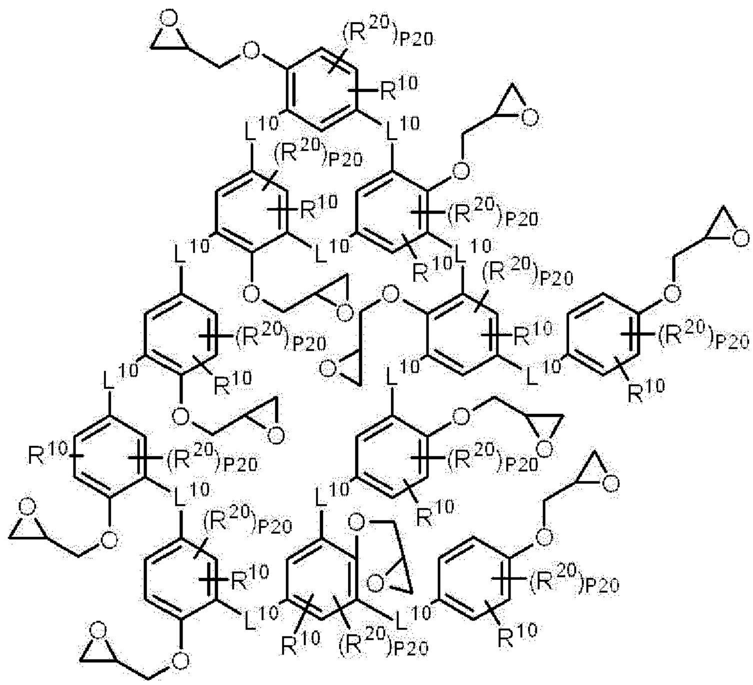

In the above structural formula, $R^{10}$, $R^{20}$ and P20 have the same meaning with $R^1$, $R^2$ and P2 in the general formula (CDN1-e), respectively. $L^{10}$ represents a single bond or a divalent linking group. A plurality of $R^{10}$s may be the same group or different groups, a plurality of $R^{20}$s may be the same group or different groups, and a plurality of $L^{10}$s may be the same group or different groups. A plurality of P20s may be the same number or different numbers.

In the above structural formula, examples of the divalent linking group represented by $L^{10}$ include an alkylene group which may have a substituent (preferably an alkylene group having 2 or more and 30 or less carbon atoms, and more preferably an alkylene group having 5 or more and 20 or less carbon atoms).

Examples of the above substituent include those listed as the substituent of $R^1$ in the general formula (CDN1).

From the viewpoint of improving the transparency of a resin molded product, the cardanol compound preferably contains a cardanol compound having an epoxy group, and is more preferably a cardanol compound having an epoxy group.

As the cardanol compound, a commercially available product may be used. Examples of the commercially available product include: NX-2024, Ultra LITE 2023, NX-2026, GX-2503, NC-510, LITE 2020, NX-9001, NX-9004, NX-9007, NX-9008, NX-9201, and NX-9203, manufactured by Cardolite Corporation; and LB-7000, LB-7250, and CD-5L, manufactured by Tohoku Chemical Industry Co., Ltd. Examples of the commercially available product of the cardanol compound having an epoxy group include NC-513, NC-514S, NC-547, LITE 513E, and Ultra LTE 513 manufactured by Cardolite Corporation.

The cardanol compound preferably has a hydroxyl value of 100 mgKOH/g or more, more preferably 120 mgKOH/g or more, and still more preferably 150 mgKOH/g or more, from the viewpoint of improving the biodegradation rate of the resin molded product. The hydroxyl value of the cardanol compound is measured according to Method A of ISO14900.

When a cardanol compound having an epoxy group is used as the cardanol compound, an epoxy equivalent is preferably 300 or more and 500 or less, more preferably 350 or more and 480 or less, and still more preferably 400 or more and 470 or less, from the viewpoint of improving the transparency of the resin molded product. The epoxy equivalent of the cardanol compound having an epoxy group is measured according to ISO3001.

The molecular weight of the cardanol compound is preferably 250 or more and 1000 or less, more preferably 280 or more and 800 or less, and still more preferably 300 or more and 500 or less, from the viewpoint of improving the biodegradation rate of the resin molded product.

The cardanol compound may be used alone or in combination of two or more thereof.

Hereinafter, the fatty acid ester (B2) will be described.

The fatty acid ester (B2) may be any of a monoester, a diester, a triester and a polyester.

Examples of the fatty acid ester (B2) include aliphatic monocarboxylic acid esters (acetate ester, etc.), aliphatic dicarboxylic acid esters (succinate ester, adipate ester, azelate ester, sebacate ester, stearate ester, etc.), aliphatic tricarboxylic acid esters (citrate ester, isocitrate ester, etc.), epoxidized fatty acid esters (epoxidized soybean oil, epoxidized flaxseed oil, epoxidized rapeseed fatty acid isobutyl, and epoxidized fatty acid 2-ethylhexyl), fatty acid methyl

esters, and sucrose esters.

The fatty acid ester may be acylated with an alkylcarboxylic acid anhydride (for example, a linear or branched alkylcarboxylic acid anhydride having 2 or more and 6 or less (preferably 2 or more and 3 or less) carbon atoms such as acetic anhydride, propionic anhydride, butyric anhydride, and valeric anhydride).

Preferred examples of the fatty acid ester (B2) include aliphatic dicarboxylic acid esters (particularly adipate ester and sebacate ester) and aliphatic tricarboxylic acid esters (particularly citrate ester).

Specific examples of the adipate ester include an adipic acid diester represented by the following general formula (AE) and an adipic acid polyester represented by the following general formula (APE).

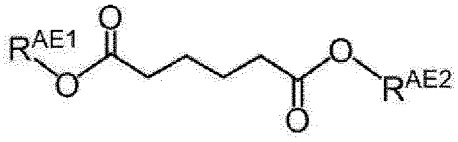

general formula (AE)

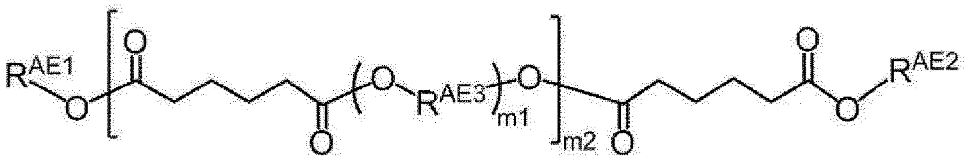

general formula (APE)

In the general formula (AE), $R^{AE1}$ and $R^{AE2}$ each independently represent an alkyl group or a polyoxyalkyl group $[-(C_xH_{2x}-O)_y-R^{A1}]$, in which $R^{A1}$ represents an alkyl group, x represents an integer of 1 or more and 10 or less, and y represents an integer of 1 or more and 10 or less.

In the general formula (APE), $R^{AE1}$ and $R^{AE2}$ each independently represent an alkyl group or a polyoxyalkyl group $[-(C_xH_{2x}-O)y-R^{A1}]$, in which $R^{A1}$ represents an alkyl group, x represents an integer of 1 or more and 10 or less, and y represents an integer of 1 or more and 10 or less, and $R^{AE3}$ represents an alkylene group. m1 represents an integer of 1 or more and 10 or less, and m2 represents an integer of 1 or more and 20 or less.

In the general formulas (AE) and (APE), the alkyl group represented by $R^{AE1}$ and $R^{AE2}$ is preferably an alkyl group having 1 or more and 12 or less carbon atoms, more preferably an alkyl group having 4 or more and 10 or less carbon atoms, and still more preferably an alkyl group having 8 carbon atoms. The alkyl group represented by $R^{AE1}$ and $R^{AE2}$ may be linear, branched or cyclic, and is preferably linear or branched.

In the general formulas (AE) and (APE), in the polyoxyalkyl group $[-(C_xH_{2x}-O)y-R^{A1}]$ represented by $R^{AE1}$ and $R^{AE2}$, the alkyl group represented by $R^{A1}$ is preferably an alkyl group having 1 or more and 6 or less carbon atoms, and more preferably an alkyl group having 1 or more and 4 or less carbon atoms. The alkyl group represented by $R^{A1}$ may be linear, branched or cyclic, and is preferably linear or branched.

In the general formula (APE), the alkylene group represented by $R^{AE3}$ is preferably an alkylene group having 1 or more and 6 or less carbon atoms, and more preferably an alkylene group having 1 or more and 4 or less carbon atoms. The alkylene group may be linear, branched or cyclic, and is preferably linear or branched.

In the general formula (APE), m1 is preferably an integer of 1 or more and 5 or less, and m2 is preferably an integer of 1 or more and 10 or less.

In the general formulas (AE) and (APE), the group represented by each reference numeral may be substituted with a substituent. Examples of the substituent include an alkyl group, an aryl group, and a hydroxy group.

As the adipate ester, a mixture of the adipate ester and other components may be used. Examples of commercially available products of the mixture include Daifatty 101 manufactured by DAIHACHI CHEMICAL INDUSTRY CO., LTD.

Examples of the sebacate ester include alkyl esters of sebacic acid having 1 or more and 12 or less (preferably 1 or more and 8 or less) carbon atoms.

Examples of the citrate ester include alkyl esters of citric acid having 1 or more and 12 or less (preferably 1 or more and 8 or less) carbon atoms. The citrate ester may be a citrate ester acylated with an alkylcarboxylic acid anhydride (for example, a linear or branched alkylcarboxylic acid anhydride having 2 or more and 6 or less (preferably 2 or more and 3 or less) carbon atoms such as acetic anhydride, propionic anhydride, butyric anhydride, and valeric anhydride).

The fatty acid ester (B2) has a molecular weight (or weight average molecular weight) of preferably 200 or more and 2000 or less, more preferably 250 or more and 1500 or less, and still more preferably 280 or more and 1000 or less. Unless otherwise specified, the weight average molecular weight of the fatty acid ester (B2) is a value measured

according to the method for measuring the weight average molecular weight of the cellulose acylate.

The content of the aromatic compound, the content of the fatty acid ester, or the total content of the aromatic compound and the fatty acid ester is preferably 1 mass% or more and 50 mass% or less, and more preferably 1 mass% or more and 30 mass% or less with respect to the cellulose resin.

The base particle may contain a resin other than the biodegradable resin. When other resins are contained, the content of other resins is preferably 5 mass% or less, and preferably less than 1 mass% with respect to the total amount of the resin composition. It is more preferable that other resins are not contained in the base particle (that is, 0 mass%).

Examples of other resins include thermoplastic resins known in the related art. Specific examples thereof include: a polycarbonate resin; a polypropylene resin; a polyester resin; a polyolefin resin; a polyester carbonate resin; a polyphenylene ether resin; a polyphenylene sulfide resin; a polysulfone resin; a polyether sulfone resin; a polyarylene resin; a polyetherimide resin; a polyacetal resin; a polyvinyl acetal resin; a polyketone resin; a polyetherketone resin; a polyetheretherketone resin; a polyarylketone resin; a polyethernitrile resin; a liquid crystal resin; a polybenzimidazole resin; a polyparavanic acid resin; a vinyl polymer or copolymer obtained by polymerizing or copolymerizing one or more vinyl monomers selected from the group consisting of an aromatic alkenyl compound, a methacrylate ester, an acrylate ester, and a vinyl cyanide compound; a diene-aromatic alkenyl compound copolymer; a vinyl cyanide-diene-aromatic alkenyl compound copolymer; an aromatic alkenyl compound-diene-vinyl cyanide-N-phenylmaleimide copolymer; a vinyl cyanide-(ethylene-diene-propylene(EPDM))-aromatic alkenyl compound copolymer; a vinyl chloride resin; and a chlorinated vinyl chloride resin. These resins may be used alone or in combination of two or more thereof.

[First Layer]

The first layer is a resin layer on the surface of the base particle. The first layer contains at least one cationic resin of a polyalkyleneimine, a polyallylamine, and a polyvinyl amine.

The cationic resin may be any of a polyalkyleneimine, a polyallylamine, and a polyvinyl amine, but a polyalkyleneimine is preferred from the viewpoints of improving the biodegradation rate over time and slowing down the initial biodegradation rate.

From the viewpoints of improving the biodegradation rate over time and slowing down the initial biodegradation rate, the polyalkyleneimine is preferably a polyalkyleneimine having a constituent unit having an alkylene group having 1 or more and 6 or less carbon atoms (preferably 1 or more and 4 or less carbon atoms, and more preferably 1 or more and 2 or less carbon atoms), and more preferably a polyethyleneimine.

In particular, the polyethyleneimine is a compound having high adhesion and high water absorbency. This is because the amino group of the polyethyleneimine has a hydrogen bond with a hydroxy group, an ionic bond with a carboxyl group, and a covalent bond with a carbonyl group. This is because the polyethyleneimine has a polar group (amino group) and a hydrophobic group (ethylene group) in the structure thereof, and therefore has a property of easily binding different substances.

In addition, the polyethyleneimine is a compound having high cationicity. Accordingly, the polyethyleneimine is present as a polycation in water and neutralizes and adsorbs anionic substances.

Further, the polyethyleneimine is a compound having high reactivity because of having a highly reactive primary amino group or secondary amino group. Accordingly, the polyethyleneimine easily reacts with various compounds.

Therefore, when the polyethyleneimine is applied to the polyalkyleneimine, the base particle is more firmly coated with the second layer containing a hydrophobic compound, and the initial biodegradation rate tends to be slowed down while the biodegradation rate over time is obtained.

The number average molecular weight of the cationic resin is preferably 300 or more and 100,000 or less, more preferably 10,000 or more and 85,000 or less, and still more preferably 50,000 or more and 80,000 or less, from the viewpoints of improving the biodegradation rate over time and slowing down the initial biodegradation rate.

The number average molecular weight of the cationic resin is measured by gel permeation chromatography (GPC apparatus: HLC-8320GPC manufactured by Tosoh Corporation, column: TSKgel$\alpha$-M) in terms of polystyrene using tetrahydrofuran.

[Second Layer]

The second layer is a compound layer on the first layer. The second layer contains an anionic or nonionic hydrophobic compound.

Examples of the anionic or nonionic hydrophobic compound include a hydrophobic compound having an anionic group (-COOH (carboxyl group), $-SO_3H$ (sulfon group), etc.), and a hydrophobic compound having no cationic or anionic group.

The hydrophobic compound refers to a compound that imparts hydrophobicity (specifically, a water contact

angle) to the biodegradable resin particle described later.

Examples of the hydrophobic compound include a silicone compound, a hydrocarbon compound, a fatty acid compound, an acrylic resin, a polyester resin, and a urethane resin.

Among these, at least one selected from the group consisting of a silicone compound, a hydrocarbon compound, a fatty acid compound, an acrylic resin, a polyester resin, and a urethane resin is preferred from the viewpoints of improving the biodegradation rate over time and slowing down the initial biodegradation rate.

Examples of the silicone compound include dimethylpolysiloxane, methylpolysiloxane, octamethylcyclotetrasiloxane, decamethylcyclopentanesiloxane, methylcyclopolysiloxane, various modified silicone oils (an alkyl-modified silicone oil, a polyether-modified silicone oil, an alcohol-modified silicone oil, a fluorine-modified silicone oil, an amino-modified silicone oil, etc.), MQ resin, and silicone rubber.

Among these, the silicone compound is preferably at least one selected from the group consisting of dimethylpolysiloxane, methylpolysiloxane, MQ resin, and silicone rubber from the viewpoints of improving the biodegradation rate over time and slowing down the initial biodegradation rate.

Here, the MQ resin refers to a silicone resin having an M unit which is a monofunctional siloxane unit $[(CH_3)_3SiO_{1/2}]$ and a Q unit which is a tetrafunctional siloxane unit $[SiO_{4/2}]$.

Examples of the commercially available product of the silicone compound include silicone compounds (KM-902, KM-903, KM-910, KM-9729, POLON-MN-ST, KM-9737A, KM-9782, KM-9738A, KM-752T, POLON-MF-33, KM-9717, X-51-1302M (MQ resin), POLON-MF-56, KM-2002-L-1, KM-2002-T, KM-9772, KM-9749, POLON-MF-40, KM-9729, X-52-1133, etc.) manufactured by Shin-Etsu Chemical Co., Ltd., and silicone compounds (BELSII, DM3112VP) manufactured by Wacker Asahikasei Silicone Co., Ltd.

Examples of the hydrocarbon compound include petroleum wax (paraffin wax, microcrystalline wax, petrolatum wax, etc.), and synthetic hydrocarbon wax (polyethylene wax, polypropylene wax, polybutene wax, fischer-tropsch wax, etc.).

Among these, the hydrocarbon compound is preferably at least one selected from the group consisting of paraffin wax, microcrystalline wax, polyethylene wax, and polypropylene wax, from the viewpoints of improving the biodegradation rate over time and slowing down the initial biodegradation rate.

Examples of the commercially available product of the hydrocarbon compound include micro-crystallin (EMUSTAR-0001, etc.) manufactured by Nippon Seiro Co., Ltd., paraffin wax (EMUSTAR-0135, etc.) manufactured by Nippon Seiro Co., Ltd., paraffin wax (AQUACER497 etc.) manufactured by BYK Co., Ltd., polyethylene wax (AQUACER507, AQUACER840, AQUACER1547, AQUACER272, etc.) manufactured by BYK Co., Ltd., polyethylene wax (Hi-Tech E-2213, Hi-Tech E-6324, etc.) manufactured by TOHO Chemical Industry Co., Ltd., polypropylene wax (AQUACER593, etc.) manufactured by BYK Co., Ltd., and polypropylene (Hi-Tech P-9018, Hi-Tech P-5060P, etc.) manufactured by TOHO Chemical Industry Co., Ltd.

Examples of the fatty acid compound include vegetable oil containing fatty acids (castor oil, tung oil, flaxseed oil, shortening, corn oil, soybean oil, sesame oil, rapeseed oil, sunflower oil, rice bran oil, camellia oil, coconut oil, palm oil, walnut oil, olive oil, peanut oil, almond oil, jojoba oil, cacao butter, shea butter, neem oil, safflower oil, japan wax, candelilla wax, rice wax, carnauba wax, etc.).

Among these, at least one selected from the group consisting of carnauba wax, rice wax, candelilla wax, palm wax, castor oil wax, soybean oil wax, and sunflower oil wax is preferred from the viewpoints of improving the biodegradation rate over time and slowing down the initial biodegradation rate.

Examples of the commercially available product of the fatty acid compound include carnauba wax (EMUSTAR-0413 (carnauba wax)) manufactured by Nippon Seiro Co., Ltd., rice wax (AQUASPROUT-7300, etc.) manufactured by Nippon Seiro Co., Ltd., palm wax (AQUASPROUT-7100, etc.) manufactured by Nippon Seiro Co., Ltd., castor oil wax (AQUASPROUT-7500, etc.) manufactured by Nippon Seiro Co., Ltd., soybean oil wax (AQUASPROUT-7200, etc.) manufactured by Nippon Seiro Co., Ltd., sunflower oil wax (AQUASPROUT-7400, etc.) manufactured by Nippon Seiro Co., Ltd., and palm oil wax (Cuckoo Ace TKE, etc.) manufactured by Nippon Seiro Co., Ltd.

Examples of the acrylic resin include well-known acrylic resins such as a polymer of acrylic acid and a polymer of an acrylic acid alkyl ester.

Examples of the commercially available product of the acrylic resin include acrylic resins (3WX-2015, 3MF-320, 3MF-333, 3MF-407, etc.) manufactured by TAISEI FINE CHEMICAL CO., LTD., and acrylic resins (COAT SFC-6440, VONCOAT CE-6270, VONCOAT CE-6400, VONCOAT CF-2800, etc.) manufactured by DIC Corporation.

Examples of the polyester resin include well-known polyester resins such as a polycondensate of a polyhydric alcohol and a polyhydric alcohol and a ring-opening polycondensate of cyclic lactam.

Examples of the commercially available product of the polyester resin include polyester resins (A-110F, A-160P, A-520, A-613D, A-615GE, A-640, A-645GH, A-647GEX, etc.) manufactured by Takamatsu Oil & Fat Co., Ltd.

Examples of the urethane resin include well-known urethane resins such as a polyester-based polyurethane, a polyether-based polyurethane, and a polycarbonate-based polyurethane. Further, as the urethane resin, a material having a urethane polymer shell layer around the acrylic polymer core may be used.

Examples of the commercially available product of the urethane resin include urethane resins (WEM-031U, WEM-200U, WEM-321U, WEM-3000, WBR-016U, WBR-2101, etc.) by TAISEI FINE CHEMICAL CO., LTD.

[Content of Each Layer]

In the biodegradable resin particle according to the present exemplary embodiment, the mass ratio of the content of the cationic resin in the first layer to the content of the hydrophobic compound in the second layer (content of cationic resin/ content of hydrophobic compound) is preferably 0.05 or more and 20 or less, more preferably 0.1 or more and 10 or less, and still more preferably 0.1 or more and 3 or less, from the viewpoints of improving the biodegradation rate over time and slowing down the initial biodegradation rate.
In addition, the content of the cationic resin with respect to the base particle is preferably 0.05 mass% or more and 15 mass% or less, more preferably 0.1 mass% or more and 10 mass% or less, and still more preferably 0.1 mass% or more and 3 mass% or less, from the viewpoints of improving the biodegradation rate over time and slowing down the initial biodegradation rate.
Further, the content of the hydrophobic compound with respect to the base particle is preferably 0.05 mass% or more and 15 mass% or less, more preferably 0.1 mass% or more and 10 mass% or less, and still more preferably 0.1 mass% or more and 3 mass% or less, from the viewpoints of improving the biodegradation rate over time and slowing down the initial biodegradation rate.
Here, each content of the cationic resin and the hydrophobic compound (that is, a content of the cationic resin in the first layer and a content of the hydrophobic compound in the second layer) is measured as follows. The content of the cationic resin is determined by the difference between the treated amount of the cationic resin and the cationic resin obtained by drying the supernatant after the treatment. Similarly, the content of the hydrophobic compound is determined by the difference between the treated amount of the hydrophobic compound and the hydrophobic compound obtained by drying the supernatant after the treatment.

[Properties of Biodegradable Resin Particle]

In the biodegradable resin particle according to the present exemplary embodiment, the water contact angle of a pellet in which the biodegradable resin particle is pelletized is 70° or more and 120° or less.
When the water contact angle is within the above range, the initial biodegradation rate is slowed down.
The water contact angle is preferably 72° or more and 110° or less, and more preferably 75° or more and 105° or less.
The water contact angle is measured 1 minute after pelletizing the prepared biodegradable resin particles and dropping 1 μl of water droplets on the surface of the pellets with a syringe using a contact angle meter (model number: CA-X type manufactured by Kyowa Interface Science Co., Ltd.) in an environment of 23°C.
The biodegradable resin particle according to the present exemplary embodiment has an aerobic condition biodegradation rate of 20% or less after 3 months as measured by a method according to ISO-14855-2 (2018).
When the aerobic condition biodegradation rate after 3 months is within the above range, the initial biodegradation rate is slowed down.
The aerobic condition biodegradation rate after 3 months is preferably 15% or less, more preferably 10% or less, and still more preferably 5% or less. The lower limit is ideally 0%, but is, for example, 1%.
In the biodegradable resin particle according to the present exemplary embodiment, the ratio of the aerobic condition biodegradation rate after 6 months to the aerobic condition biodegradation rate after 3 months (aerobic condition biodegradation rate after 6 months/aerobic condition biodegradation rate after 3 months) as measured by the method according to ISO-14855-2 (2018) is preferably 1.20 or more, preferably 1.50 or more, and more preferably 2.00 or more.
In addition, the ratio of the aerobic condition biodegradation rate after 12 months to the aerobic condition biodegradation rate after 3 months (aerobic condition biodegradation rate after 12 months/aerobic condition biodegradation rate after 3 months) as measured by the method according to ISO-14855-2 (2018) is preferably 3.50 or more, preferably 5.00 or more, more preferably 10.00 or more, still more preferably 15.00 or more, and even more preferably 20.00 or more.
In the biodegradable resin particle according to the present exemplary embodiment, when the surface of the biodegradable resin particle is measured by X-ray photoelectron spectroscopy (XPS), a relationship between a carbon atomic weight Cs, a silicon atomic weight Sis, and an oxygen atomic weight Os satisfies an expression A: $(Cs + Sis)/Os \geq 3$.
When the expression A is satisfied, the initial biodegradation rate is slowed down. In addition, the oil lipophilicity of the surface of the biodegradable resin particle is improved, and the oil absorption rate of the resin particle is increased.
The value of "(Cs + Sis)/Os" is preferably 4 or more, and more preferably 7 or more, from the viewpoint of reducing the initial biodegradation rate and improving the oil absorption rate. However, the upper limit of the value of "(Cs + Sis)/Os" is, for example, 95 from the viewpoint of the biodegradation rate over time.

In order to make the value of "(Cs + Sis)/Os" within the above range, it is preferable to coat the base particle with the second layer containing a hydrophobic compound.

In the biodegradable resin particle according to the present exemplary embodiment, when the surface of the biodegradable resin particle is measured by X-ray photoelectron spectroscopy (XPS) 3 minutes after surface etching, a relationship between a carbon atomic weight Ce, a silicon atomic weight Sie, and an oxygen atomic weight Oe preferably satisfies an expression B: $(Ce + Sie)/Oe > 3$.

When the expression B is satisfied, the period during which the biodegradation rate is reduced becomes longer. In addition, a high oil absorption rate can be easily maintained. Further, even when a mechanical load is applied to the biodegradable resin particle (for example, even when the biodegradable resin particle is agitated by ultrasonic homogenization or the like), the oil absorption rate is unlikely to decrease.

The value of "(Ce + Sie)/Oe" is more preferably 4 or more, and still more preferably 7 or more, from the viewpoint of the initial biodegradation rate maintenance and the oil absorption rate maintenance. However, the upper limit of the value of "(Ce + Sie)/Oe" is, for example, 95 from the viewpoint of the biodegradation rate over time.

In order to make the value of "(Ce + Sie)/Oe" within the above range, it is preferable to sequentially coat the base particle with the first layer containing a cationic resin and the second layer containing a hydrophobic compound.

Here, the method of measuring each atomic weight (atom%) by X-ray photoelectron spectroscopy (XPS) is as follows. The measurement is performed on the prepared biodegradable resin particle.

"PHI5000 Versa Probe II manufactured by ULVAC-PHI Inc." is used as an XPS measurement device, monochromatic AlK$\alpha$ rays are used as an X-ray source, and the acceleration voltage is set to 15 kV for measurement. Specifically, assuming that the analysis region is 100 $\mu$m$\varphi$, the number of each atom (the carbon atom, the silicon atom and the oxygen atom) is obtained based on the spectrum of each measured atom, and each atomic weight (the carbon atomic weight, the silicon atomic weight, and the oxygen atomic weight) with respect to the total atomic weight in the measurement region is calculated.

Then, the carbon atomic weight, the silicon atomic weight and the oxygen atomic weight when the surface of the biodegradable resin particle before etching is measured are obtained as the carbon atomic weight Cs, the silicon atomic weight Sis and the oxygen atomic weight Os.

In addition, the carbon atomic weight, the silicon atomic weight and the oxygen atomic weight when the surface of the biodegradable resin particle 3 minutes after the surface etching are measured are obtained as the carbon atomic weight Ce, the silicon atomic weight Sie and the oxygen atomic weight Oe.

However, when the compound contained in the second layer does not contain a silicon atom, the silicon atomic weight is 0 atom%.

The surface etching is performed on the prepared biodegradable resin particle as follows.

Using "PHI5000 Versa Probe II manufactured by ULVAC-PHI Inc." as an etching device and using an argon gas cluster etching method using argon as an etching gas, etching is performed for 3 minutes under the conditions of an acceleration voltage of 5 kV and a sweep region of 2 mm $\times$ 2 mm.

The volume average particle diameter of the biodegradable resin particle is preferably 3 $\mu$m or more and 100 $\mu$m or less, more preferably 5 $\mu$m or more and 70 $\mu$m or less, and still more preferably 8 $\mu$m or more and 60 $\mu$m or less.

When the particle diameter of the biodegradable resin particle is 3 $\mu$m or more, the number of particles per unit weight does not become too large, so that the decrease in the biodegradation rate is prevented. On the other hand, when the particle diameter of the biodegradable resin particle is 100 $\mu$m or less, the specific surface area is increased and the biodegradation rate can be further improved.

Therefore, the volume average particle diameter of the biodegradable resin particle is preferably within the above range.

The large-diameter side particle size distribution index GSDv of the biodegradable resin particle is preferably 1.5 or less, more preferably 1.3 or less, and still more preferably 1.2 or less.

When the particle size distribution of the biodegradable resin particle is brought close to uniform, regular hydrolysis proceeds by giving a certain contact opportunity with water, and the biodegradation rate can be further improved.

The volume average particle diameter and the large-diameter-side particle size distribution index GSDv of the biodegradable resin particle are measured as follows.

The particle diameter is measured with an LS particle size distribution measuring device "Beckman Coulter LS13 320 (manufactured by Beckman Coulter, Inc.)", the cumulative distribution of the particle diameter is drawn from the small diameter side on a volume basis, and the particle diameter at which the cumulation is 50% is determined as the volume average particle diameter.

On the other hand, the cumulative distribution of the particle diameter is drawn from the small diameter side on a volume basis, and the particle diameter corresponding to the cumulative percentage of 50% is defined as a number average particle diameter D50v, and the particle diameter corresponding to the cumulative percentage of 84% is defined as a number particle diameter D84v. Then, the large-diameter-side particle size distribution index GSDv is calculated

according to the equation $GSDv = (D84v/D50v)^{1/2}$.

[Method for Producing Biodegradable Resin Particle]

Examples of the method for producing the biodegradable resin particle according to the present exemplary embodiment include a method including:

a first step of mixing an aqueous dispersion, in which base particles are dispersed, with an aqueous solution containing a cationic resin; and
a second step of taking out the base particles from the mixed solution, obtaining an aqueous dispersion in which the base particles are dispersed, mixing the aqueous dispersion with an emulsion solution of an anionic or nonionic hydrophobic compound, and then drying the mixture.

Hereinafter, a specific description will be given.

-First Step-

In the first step, the base particles are prepared.
Examples of the method for producing the base particles include the following methods.

1) a kneading and crushing method in which each component is kneaded and the obtained kneaded product is crushed and classified to obtain granules
2) a dry process method in which the granules obtained by the kneading and crushing method are changed in shape by mechanical impact force or thermal energy to obtain granules
3) an agglomeration and coalescence method in which particle dispersions of respective components are mixed, and particles in the dispersion are agglomerated and heat-fused to obtain granules
4) a dissolution and suspension method in which an organic solvent in which each component is dissolved is suspended in an aqueous solvent to granulate granules containing each component

Among these, wet methods such as the agglomeration and coalescence method and the dissolution and suspension method are preferred from the viewpoints of obtaining a biodegradable resin particle having a volume average particle diameter and a large-diameter side particle size distribution index GSDv to be described later.
Next, an aqueous dispersion in which the obtained base particles are dispersed is prepared. It is preferable to acid-wash the base particles before preparing the aqueous dispersion.
Next, the aqueous dispersion in which the base particles are dispersed, and an aqueous solution containing a cationic resin are mixed. Thereby, for example, the hydroxy group of the resin contained in the base particles reacts with the amine site of the cationic resin to form the first layer.

-Second Step-

In the second step, the base particles on which the first layer is formed are taken out from the mixed solution. Taking out the base particles is carried out, for example, by filtering the mixed solution. The base particles taken out may be washed with water. Thereby, the unreacted cationic resin can be removed.
Next, an aqueous dispersion in which the base particles are dispersed is prepared, and then the aqueous dispersion and an emulsion solution of an anionic or nonionic hydrophobic compound are mixed. Thereby, the emulsion of the hydrophobic compound is adsorbed on the first layer of the base particles.
Thereafter, when the mixed solution is dried, the emulsion of the hydrophobic compound is broken, and the hydrophobic compound is filmed on the first layer. Thereby, the second layer is formed.
With the above steps, the biodegradable resin particle according to the present exemplary embodiment can be obtained.
Examples of the uses of the biodegradable resin particle according to the present exemplary embodiment include granules of cosmetic base materials, rolling agents, abrasives, scrubbing agents, display spacers, bead molding materials, light diffusing particles, resin strengthening agents, refractive index control agents, biodegradation accelerators, fertilizers, water-absorbent particles, and toner particles.

Examples

Hereinafter, a resin composition and a resin molded product according to the present exemplary embodiment

will be described in more detail with reference to Examples. The materials, amounts used, proportions, treatment procedures, etc. shown in the following Examples can be appropriately changed within a range not deviate from the gist of the present invention. Therefore, the resin composition and the resin molded product according to the present exemplary embodiment should not be construed in a limited manner by the specific examples shown below.

<Preparation of Each Material>

The following materials are prepared.

[Biodegradable Resin of Base Particle]

- DAC: "L-50" manufactured by Daicel Corporation, cellulose diacetate, weight-average degree of polymerization: 570
- CAB: "CAP504-0.2" manufactured by Eastman Chemical Company, cellulose acetate propionate, weight-average degree of polymerization: 133, degree of acetyl group substitution: 0.04, degree of propionyl group substitution: 2.09
- CAP: cellulose acetate propionate, weight-average degree of polymerization: 716, degree of acetyl group substitution: 0.18, degree of propionyl group substitution: 2.49
- PLA: polylactic acid, weight average molecular weight: 180,000
- PHA: polyhydroxyalkanoic acid
- PBS: polybutylene succinate, weight average molecular weight: 200,000
- PBSA: polybusylene succinate adipate, weight average molecular weight: 110,000
- PBAT: polybutylene adipate terephthalate, weight average molecular weight: 100,000
- PETS: polyethylene terephthalate/succinate copolymer, weight average molecular weight: 150,000

[Plasticizer of Base Particle]

- CDN1: "NX-2503" manufactured by Cardolite Corporation, hydroxyethylated cardanol, molecular weight: 296 to 320
- CDN2: "Ultra LITE 513" manufactured by Cardolite Corporation, cardanol glycidyl ether, molecular weight: 354 to 361
- CDN3: "EPICLON865-alkyl-modified product" manufactured by DIC Corporation, alkyl-modified product of phenol novolac type epoxy resin
- DBA: diisobutyl adipate
- ATBC: triethyl O-acetyl citrate
- DPS: diisopropyl sebacate

[Cationic Resin of First Layer]

- PEI: polyethylenimine, the number average molecular weight Mn of the material used is shown in Table 1.

- PAA: polyallylamine, the number average molecular weight Mn of the material used is shown in Table 1.

- PVAM: polyvinylamine, the number average molecular weight Mn of the material used is shown in Table 1.

[Anionic or Nonionic Hydrophobic Compound of Second Layer]

- EMUSTAR-0135: paraffin wax manufactured by Nippon Seiro Co., Ltd.
- POLON-MN-ST: dimethyl silicone manufactured by Shin-Etsu Chemical Co., Ltd.
- KM-9717: MQ resin manufactured by Shin-Etsu Chemical Co., Ltd.
- BELSIL DM3112VP: dimethicone (dimethyl silicone) manufactured by Wacker Asahikasei Silicone Co., Ltd.
- Hi-Tech E-2213: polyethylene wax manufactured by TOHO Chemical Industry Co., Ltd.
- Hi-Tech P-9018: polypropylene wax manufactured by TOHO Chemical Industry Co., Ltd.

- EMUSTAR-0413: carnauba wax manufactured by Nippon Seiro Co., Ltd.
- 3MF-320: acrylic resin manufactured by TAISEI FINE CHEMICAL CO., LTD.
- A-647GEX: polyester resin manufactured by Takamatsu Oil & Fat Co., Ltd.
- WBR-016U: Urethane resin manufactured by TAISEI FINE CHEMICAL CO., LTD.

[Examples A1 to A30, B 1 to B29, Comparative Examples A1 to A2, B 1 to B2]

(Preparation of Resin Pellet)

According to the charged composition ratios shown in Tables 1 and 2, the cylinder temperature is adjusted, and kneading is carried out with a twin-screw kneader (TEX41SS manufactured by Toshiba Machine Co., Ltd.) to obtain a pellet-shaped resin composition (hereinafter referred to as a resin pellet).

(Preparation of Base Particle)

When DAC, CAB, and CAP are used as the resin, the base particles are obtained as follows.

300 g of the resin pellet is completely dissolved in 700 g of methyl ethyl ketone. The mixture is added to an aqueous liquid in which 100 g of calcium carbonate, 4 g of carboxymethyl cellulose and 200 g of methyl ethyl ketone are dispersed in 1100 g of pure water, and the mixture is stirred for 3 hours. 10 g of sodium hydroxide is added thereto, and the mixture is heated to 80°C and stirred for 3 hours to remove methyl ethyl ketone. After filtering the residue, the obtained substance is dispersed in pure water again to obtain a slurry of base particles.

On the other hand, when a resin other than DAC, CAB, and CAP is used as the resin, the base particles are obtained as follows.

2000 g of the resin pellet is melt-kneaded (kneader), the kneaded product is rolled with two rolls to form a plate, and then the molded product is cooled and roughly pulverized by a crusher. The roughly crushed product is finely crushed with a jet mill, to obtain base particles. The base particles are dispersed in pure water to obtain a slurry of base particles.

(Preparation of Biodegradable Resin Particle)

Using the materials of the first layer and the second layer shown in Tables 1 and 2, biodegradable resin particles are obtained as follows such that the content in each layer is the amount shown in Tables 1 and 2.

After adjusting the slurry of base particles to have a solid content of 20%, a predetermined amount of a cationic resin solution is added with respect to the amount of solid content contained in this slurry in terms of pure content, and stirring is performed under the condition of 25°C for 1 hour. After the stirring is completed, the residue is filtered and the obtained substance is dispersed in pure water again to adjust the solid content to 20%, a predetermined amount of a hydrophobic compound is added with respect to the amount of solid content contained in this slurry in terms of pure content, and stirring is performed under the condition of 25°C for 1 hour. After the stirring is completed, the residue is filtered and the solid content is freeze-dried to obtain biodegradable resin particles.

Through the above steps, biodegradable resin particles are obtained.

In Comparative Examples A2 and B2, the treatment of forming the second layer (addition treatment of the hydrophobic compound) is carried out to have the same content as in Examples A1 and B 1 without forming the first layer, but the hydrophobic compound is difficult to adsorb, and the content in the second layer is 0.0001%.

<Properties of Biodegradable Resin Particle>

The following properties of the biodegradable resin particle are measured according to the methods described. The results are shown in Tables 1 and 2.

- Water contact angle (written as "contact angle" in the table)
- Volume average particle diameter D50v
- (Cs + Sis)/Os value (written as "(C + Si)/O ratio, T = 0" in the table)
- (Ce + Sie)/Oe value (written as "(C + Si)/O ratio, T = 3" in the table)

<Hydrolysis Resistance>

The hydrolysis resistance of the biodegradable resin particle is evaluated as follows. However, the evaluation of hydrolysis resistance is carried out only for the biodegradable resin particles of Examples B1 to B29 and Comparative Examples B1 to B2.

50 g of biodegradable resin particles are sealed in a bag made of #508/585-1 μ nylon mesh (opening 1 μm), and immersed in distilled water adjusted to a pH of 7.8 at 50°C for 90 days. Thereafter, the nylon mesh containing the resin particles is taken out, and the resin particles in the nylon mesh are vacuum dried. The mass of the biodegradable resin particles after vacuum drying is measured, and the mass reduction rate of the biodegradable resin particles is measured. Then, evaluation is performed according to the following criteria.

A: the mass reduction rate is 0% or more and less than 10%
B: the mass reduction rate is 10% or more and less than 20%
C: the mass reduction rate is 20% or more and less than 30%
D: the mass reduction rate is 30% or more

<Oil Absorbency>

-Original Oil Absorption Rate-

After 1 g of the biodegradable resin particles are dispersed in 10 g of linseed oil, centrifugation is performed at 10,000 rpm for 20 minutes to gently remove the supernatant, and then the mass of the biodegradable resin particles is measured. Then, the oil absorption rate is calculated according to the following equation.

· Equation: oil absorption rate = (mass increase after centrifugation/mass before dispersion in linseed oil) $\times$ 100

-Oil Absorption Rate after Stirring-

An ultrasonic homogenizer is placed in a solution in which 1 g of biodegradable resin particles are dispersed in 10 g of linseed oil, ultrasonic waves of 20 kHz are emitted, and the solution is stirred for 10 minutes. The solution after stirring is centrifuged at 10,000 rpm for 20 minutes to gently remove the supernatant, and then the mass of the biodegradable resin particles is measured. Then, the oil absorption rate is calculated according to the above equation.

The oil absorption rate is evaluated according to the following criteria.

(A): 110% or more
(B): 90% or more and 109% or less
(C) 70% or more and 89% or less
(D): 69% or less

<Measurement of Biodegradability>

Using the obtained biodegradable resin particles, the aerobic condition biodegradation rates after 3, 6, and 12 months are measured by the method according to ISO-14855-2 (2018). The results are shown in Tables 1 and 2.

[Table 1-1]

| | Base particles | First layer (cationic resin) | | | Second layer (hydrophobic compound) | | | Content ratio A/B | Contact angle (°) | (C + Si)/O ratio | |
|---|---|---|---|---|---|---|---|---|---|---|---|
| | Resin type | Type | Mn | Content A (%) (with respect to base particles) | Type | | Content B (%) (with respect to base particles) | | | T = 0 | T = 3 |
| Example A1 | DAC | PEI | 70000 | 1 | EMUSTAR-0135 | Paraffin wax | 1 | 1 | 101 | 18 | 9 |
| Example A2 | DAC | PEI | 70000 | 0.1 | EMUSTAR-0135 | Paraffin wax | 1 | 0.1 | 90 | 17 | 8 |
| Example A3 | DAC | PEI | 70000 | 10 | EMUSTAR-0135 | Paraffin wax | 1 | 10 | 93 | 19 | 8 |
| Example A4 | DAC | PEI | 70000 | 1 | EMUSTAR-0135 | Paraffin wax | 0.1 | 10 | 82 | 6 | 3 |
| Example A5 | DAC | PEI | 70000 | 1 | EMUSTAR-0135 | Paraffin wax | 10 | 0.1 | 103 | 50 | 35 |
| Example A6 | DAC | PEI | 300 | 1 | EMUSTAR-0135 | Paraffin wax | 1 | 1 | 75 | 13 | 5 |
| Example A7 | DAC | PEI | 100000 | 1 | EMUSTAR-0135 | Paraffin wax | 1 | 1 | 83 | 19 | 9 |
| Example A8 | CAB | PEI | 70000 | 1 | EMUSTAR-0135 | Paraffin wax | 1 | 1 | 81 | 22 | 10 |
| Example A9 | CAP | PEI | 70000 | 1 | EMUSTAR-0135 | Paraffin wax | 1 | 1 | 80 | 22 | 12 |
| Example A10 | PLA | PEI | 70000 | 1 | EMUSTAR-0135 | Paraffin wax | 1 | 1 | 78 | 5 | 3 |
| Example A11 | PBS | PEI | 70000 | 1 | EMUSTAR-0135 | Paraffin wax | 1 | 1 | 73 | 5 | 3 |
| Example A12 | PHA | PEI | 70000 | 1 | EMUSTAR-0135 | Paraffin wax | 1 | 1 | 74 | 7 | 4 |
| Example A13 | PBSA | PEI | 70000 | 1 | EMUSTAR-0135 | Paraffin wax | 1 | 1 | 76 | 7 | 4 |

(continued)

| | Base particles | First layer (cationic resin) | | | Second layer (hydrophobic compound) | | | Content ratio A/B | Contact angle (°) | (C + Si)/O ratio | |
|---|---|---|---|---|---|---|---|---|---|---|---|
| | Resin type | Type | Mn | Content A (%) (with respect to base particles) | Type | | Content B (%) (with respect to base particles) | | | T = 0 | T = 3 |
| Example A14 | PBAT | PEI | 70000 | 1 | EMUSTAR-0135 | Paraffin wax | 1 | 1 | 72 | 4 | 3 |
| Example A15 | PETS | PEI | 70000 | 1 | EMUSTAR-0135 | Paraffin wax | 1 | 1 | 72 | 4 | 3 |
| Example A16 | DAC | PEI | 70000 | 1 | POLON-MN-ST | Dimethyl silicone | 1 | 1 | 102 | 3 | 3 |
| Example A17 | DAC | PEI | 70000 | 1 | KM-9717 | MQ resin | 1 | 1 | 100 | 3 | 3 |
| Example A18 | DAC | PEI | 70000 | 1 | BELSIL DM3112VP | Dimethicone | 1 | 1 | 99 | 3 | 3 |
| Example A19 | DAC | PEI | 70000 | 1 | Hi-Tech E-2213 | Polyethylene wax | 1 | 1 | 87 | 19 | 9 |
| Example A20 | DAC | PEI | 70000 | 1 | Hi-Tech P-9018 | Polypropylene wax | 1 | 1 | 85 | 18 | 8 |
| Example A21 | DAC | PEI | 70000 | 1 | EMUSTAR-0413 | Carnauba wax | 1 | 1 | 90 | 7 | 4 |
| Example A22 | DAC | PEI | 70000 | 1 | 3MF-320 | Acrylic resin | 1 | 1 | 84 | 3.5 | 3.5 |
| Example A23 | DAC | PEI | 70000 | 1 | A-647GEX | Polyester resin | 1 | 1 | 82 | 3.5 | 3.5 |
| Example A24 | DAC | PEI | 70000 | 1 | WBR-016U | Urethane resin | 1 | 1 | 80 | 3.5 | 3.5 |
| Example A25 | DAC | PEI | 70000 | 0.05 | EMUSTAR-0135 | Paraffin wax | 1 | 0.05 | 74 | 13 | 5 |
| Example A26 | DAC | PEI | 70000 | 15 | EMUSTAR-0135 | Paraffin wax | 1 | 15 | 92 | 18 | 9 |
| Example A27 | DAC | PEI | 70000 | 1 | EMUSTAR-0135 | Paraffin wax | 0.05 | 20 | 75 | 4 | 3 |

(continued)

| | Base particles | First layer (cationic resin) | | | Second layer (hydrophobic compound) | | | Content ratio A/B | Contact angle (°) | (C + Si)/O ratio | |
|---|---|---|---|---|---|---|---|---|---|---|---|
| | Resin type | Type | Mn | Content A (%) (with respect to base particles) | Type | | Content B (%) (with respect to base particles) | | | T = 0 | T = 3 |
| Example A28 | DAC | PEI | 70000 | 1 | EMUSTAR-0135 | Paraffin wax | 15 | 0.07 | 85 | 95 | 70 |
| Example A29 | DAC | PAA | 25000 | 1 | EMUSTAR-0135 | Paraffin wax | 1 | 1 | 90 | 18 | 8 |
| Example A30 | DAC | PVAM | 100000 | 1 | EMUSTAR-0135 | Paraffin wax | 1 | 1 | 87 | 16 | 7 |
| Comparative Example A1 | DAC | PEI | 70000 | 1 | EMUSTAR-0135 | Paraffin wax | 0 | - | 60 | 2 | 2 |
| Comparative Example A2 | DAC | PEI | 70000 | 0 | EMUSTAR-0135 | Paraffin wax | 0.0001 | - | 62 | 2 | 2 |

[Table 1-2]

| | Initial oil absorption rate (%) | Oil absorption rate after stirring (%) | D50v (μm) | Biodegradability (%) | | | | |
|---|---|---|---|---|---|---|---|---|
| | | | | After 3 months | After 6 months | After 12 months | Ratio of after 6 months/ after 3 months | Ratio of after 12 months/ after 3 months |
| Example A1 | (B) 105 | (B) 95 | 10 | 3 | 10 | 93 | 3.33 | 31.00 |
| Example A2 | (B) 105 | (B) 95 | 10 | 10 | 25 | 95 | 2.50 | 9.50 |
| Example A3 | (B) 105 | (B) 95 | 10 | 5 | 13 | 89 | 2.60 | 17.80 |
| Example A4 | (B) 95 | (C) 85 | 10 | 14 | 30 | 95 | 2.14 | 6.79 |
| Example A5 | (A) 120 | (A) 110 | 10 | 2 | 7 | 82 | 3.50 | 41.00 |
| Example A6 | (B) 100 | (B) 90 | 10 | 16 | 25 | 94 | 1.56 | 5.88 |
| Example A7 | (B) 105 | (B) 95 | 10 | 4 | 9 | 84 | 2.25 | 21.00 |
| Example A8 | (B) 105 | (B) 100 | 12 | 8 | 13 | 94 | 1.63 | 11.75 |
| Example A9 | (B) 105 | (B) 100 | 8 | 11 | 18 | 95 | 1.64 | 8.64 |
| Example A10 | (C) 90 | (C) 80 | 9 | 3 | 10 | 80 | 3.33 | 26.67 |
| Example A11 | (C) 90 | (C) 80 | 8 | 12 | 24 | 82 | 2.00 | 6.83 |
| Example A12 | (C) 90 | (C) 80 | 11 | 10 | 22 | 83 | 2.20 | 8.30 |
| Example A13 | (C) 90 | (C) 80 | 10 | 15 | 25 | 85 | 1.67 | 5.67 |
| Example A14 | (C) 85 | (C) 75 | 12 | 9 | 20 | 79 | 2.22 | 8.78 |
| Example A15 | (C) 85 | (C) 75 | 9 | 10 | 23 | 76 | 2.30 | 7.60 |
| Example A16 | (C) 80 | (C) 80 | 10 | 2 | 6 | 82 | 3.00 | 41.00 |
| Example A17 | (C) 80 | (C) 80 | 10 | 2 | 5 | 83 | 2.50 | 41.50 |
| Example A18 | (C) 80 | (C) 80 | 10 | 7 | 12 | 85 | 1.71 | 12.14 |
| Example A19 | (B) 105 | (B) 95 | 10 | 3 | 8 | 82 | 2.67 | 27.33 |
| Example A20 | (B) 105 | (B) 95 | 10 | 2 | 5 | 80 | 2.50 | 40.00 |
| Example A21 | (B) 95 | (B) 90 | 10 | 10 | 23 | 96 | 2.30 | 9.60 |
| Example A22 | (C) 80 | (C) 75 | 10 | 4 | 8 | 79 | 2.00 | 19.75 |
| Example A23 | (C) 80 | (C) 75 | 10 | 6 | 10 | 80 | 1.67 | 13.33 |
| Example A24 | (C) 80 | (C) 75 | 10 | 9 | 11 | 82 | 1.22 | 9.11 |
| Example A25 | (B) 100 | (B) 90 | 10 | 17 | 34 | 97 | 2.00 | 5.71 |
| Example A26 | (B) 105 | (B) 95 | 10 | 2 | 8 | 87 | 4.00 | 43.50 |
| Example A27 | (B) 90 | (C) 80 | 10 | 20 | 32 | 95 | 1.60 | 4.75 |
| Example A28 | (A) 130 | (A) 125 | 10 | 4 | 6 | 80 | 1.50 | 20.00 |
| Example A29 | (B) 105 | (B) 95 | 10 | 12 | 20 | 93 | 1.67 | 7.75 |
| Example A30 | (B) 105 | (B) 95 | 10 | 14 | 22 | 90 | 1.57 | 6.43 |
| Comparative Example A1 | (D) 60 | (D) 60 | 10 | 30 | 45 | 98 | 1.50 | 3.27 |
| Comparative Example A2 | (D) 60 | (D) 60 | 10 | 60 | 78 | 98 | 1.30 | 1.63 |

[Table 2-1]

| | Base particles | | | | First layer (cationic resin) | | | Second layer (hydrophobic compound) | | | Content ratio A/B | Contact angle (°) | (C + Si)/O ratio | |
|---|---|---|---|---|---|---|---|---|---|---|---|---|---|---|
| | Resin | | Plasticizer | | Type | Mn | Content A (%) (with respect to base particles) | Type | | Content B (%) (with respect to base particles) | | | T = 0 | T=3 |
| | Type | Part | Type | Part | | | | | | | | | | |
| Example B1 | CAP | 100 | CDN1 | 20 | PEI | 70000 | 1 | EMUSTAR-0413 | Carnauba wax | 1 | 1 | 92 | 8 | 4 |
| Example B2 | CAP | 100 | CDN1 | 20 | PEI | 70000 | 0.1 | EMUSTAR-0413 | Carnauba wax | 1 | 0.1 | 86 | 7 | 4 |
| Example B3 | CAP | 100 | CDN1 | 20 | PEI | 70000 | 10 | EMUSTAR-0413 | Carnauba wax | 1 | 10 | 85 | 9 | 5 |
| Example B4 | CAP | 100 | CDN1 | 20 | PEI | 70000 | 1 | EMUSTAR-0413 | Carnauba wax | 0.1 | 10 | 81 | 4 | 3 |
| Example B5 | CAP | 100 | CDN1 | 20 | PEI | 70000 | 1 | EMUSTAR-0413 | Carnauba wax | 10 | 0.1 | 84 | 11 | 8 |
| Example B6 | CAP | 100 | CDN1 | 20 | PEI | 300 | 1 | EMUSTAR-0413 | Carnauba wax | 1 | 1 | 84 | 7 | 4 |
| Example B7 | CAP | 100 | CDN1 | 20 | PEI | 100000 | 1 | EMUSTAR-0413 | Carnauba wax | 1 | 1 | 80 | 8 | 4 |
| Example B8 | CAP | 100 | CDN1 | 20 | PEI | 70000 | 1 | POLON-MN-ST | Dimethyl silicone | 1 | 1 | 102 | 3 | 3 |
| Example B9 | CAP | 100 | CDN1 | 20 | PEI | 70000 | 1 | KM-9717 | MQ resin | 1 | 1 | 103 | 3 | 3 |
| Example B10 | CAP | 100 | CDN1 | 20 | PEI | 70000 | 1 | BELSIL DM3112VP | Dimethicone | 1 | 1 | 99 | 3 | 3 |
| Example B11 | CAP | 100 | CDN1 | 20 | PEI | 70000 | 1 | Hi-Tech E-2213 | Polyethylene wax | 1 | 1 | 88 | 20 | 10 |
| Example B12 | CAP | 100 | CDN1 | 20 | PEI | 70000 | 1 | Hi-Tech P-9018 | Polypropylene wax | 1 | 1 | 87 | 20 | 10 |
| Example B13 | CAP | 100 | CDN1 | 20 | PEI | 70000 | 1 | 3MF-320 | Acrylic resin | 1 | 1 | 83 | 3.5 | 3.5 |

(continued)

| | Base particles | | | | First layer (cationic resin) | | | Second layer (hydrophobic compound) | | | Content ratio A/B | Contact angle (°) | (C + Si)/O ratio | |
|---|---|---|---|---|---|---|---|---|---|---|---|---|---|---|
| | Resin | | Plasticizer | | Type | Mn | Content A (%) (with respect to base particles) | Type | | Content B (%) (with respect to base particles) | | | T = 0 | T=3 |
| | Type | Part | Type | Part | | | | | | | | | | |
| Example B14 | CAP | 100 | CDN1 | 20 | PEI | 70000 | 1 | A-647GEX | Polyester resin | 1 | 1 | 82 | 3.5 | 3.5 |
| Example B15 | CAP | 100 | CDN1 | 20 | PEI | 70000 | 1 | WBR-016U | Urethane resin | 1 | 1 | 80 | 3.5 | 3.5 |
| Example B16 | CAP | 100 | CDN1 | 20 | PEI | 70000 | 0.05 | EMUSTAR-0413 | Carnauba wax | 1 | 0.05 | 73 | 6 | 4 |
| Example B17 | CAP | 100 | CDN1 | 20 | PEI | 70000 | 15 | EMUSTAR-0413 | Carnauba wax | 1 | 15 | 75 | 9 | 5 |
| Example B18 | CAP | 100 | CDN1 | 20 | PEI | 70000 | 1 | EMUSTAR-0413 | Carnauba wax | 0.05 | 20 | 70 | 3.5 | 3 |
| Example B19 | CAP | 100 | CDN1 | 20 | PEI | 70000 | 1 | EMUSTAR-0413 | Carnauba wax | 15 | 0.07 | 78 | 15 | 12 |
| Example B20 | CAP | 100 | CDN1 | 20 | PAA | 25000 | 1 | EMUSTAR-0413 | Carnauba wax | 1 | 1 | 77 | 7 | 4 |
| Example B21 | CAP | 100 | CDN1 | 20 | PVAM | 100000 | 1 | EMUSTAR-0413 | Carnauba wax | 1 | 1 | 74 | 7 | 4 |
| Example B22 | CAB | 100 | CDN1 | 20 | PEI | 70000 | 1 | EMUSTAR-0413 | Carnauba wax | 1 | 1 | 80 | 8 | 4 |
| Example B23 | CAB | 100 | DBA | 20 | PEI | 70000 | 1 | EMUSTAR-0413 | Carnauba wax | 1 | 1 | 90 | 9 | 5 |
| Example B24 | CAB | 100 | ATB C | 20 | PEI | 70000 | 1 | EMUSTAR-0413 | Carnauba wax | 1 | 1 | 90 | 9 | 5 |
| Example B25 | CAB | 100 | DPS | 20 | PEI | 70000 | 1 | EMUSTAR-0413 | Carnauba wax | 1 | 1 | 89 | 8 | 4 |
| Example B26 | CAP | 100 | DBA | 20 | PEI | 70000 | 1 | EMUSTAR-0413 | Carnauba wax | 1 | 1 | 81 | 8 | 4 |

(continued)

| | Base particles | | | | First layer (cationic resin) | | | Second layer (hydrophobic compound) | | | Content ratio A/B | Contact angle (°) | (C + Si)/O ratio | |
|---|---|---|---|---|---|---|---|---|---|---|---|---|---|---|
| | Resin | | Plasticizer | | Type | Mn | Content A (%) (with respect to base particles) | Type | | Content B (%) (with respect to base particles) | | | T = 0 | T=3 |
| | Type | Part | Type | Part | | | | | | | | | | |
| Example B27 | CAP | 100 | CDN2 | 20 | PEI | 70000 | 1 | EMUSTAR-0413 | Carnauba wax | 1 | 1 | 82 | 8 | 4 |
| Example B28 | CAP | 100 | CDN3 | 20 | PEI | 70000 | 1 | EMUSTAR-0413 | Carnauba wax | 1 | 1 | 78 | 8 | 4 |
| Example B29 | CAP | - | - | - | PEI | 70000 | 1 | EMUSTAR-0413 | Carnauba wax | 1 | 1 | 80 | 7 | 4 |
| Comparative Example B 1 | CAP | 100 | CDN1 | 20 | PEI | 70000 | 1 | EMUSTAR-0413 | Carnauba wax | 0 | - | 60 | 2.5 | 2.5 |
| Comparative Example B2 | CAP | 100 | CDN1 | 20 | PEI | 70000 | 0 | EMUSTAR-0413 | Carnauba wax | 0.0001 | - | 63 | 2.5 | 2.5 |

[Table 2-2]

| | Initial oil absorption rate (%) | Oil absorption rate after stirring (%) | D50v (μm) | Hydrolysis resistance | Biodegradability (%) | | | | |
|---|---|---|---|---|---|---|---|---|---|
| | | | | | After 3 months | After 6 months | After 12 months | Ratio of after 6 months/ after 3 months | Ratio of after 12 months/ after 3 months |
| Example B1 | (B) 100 | (B) 95 | 7 | A | 15 | 42 | 100 | 2.80 | 6.67 |
| Example B2 | (B) 100 | (B) 95 | 7 | B | 17 | 44 | 100 | 2.59 | 5.88 |
| Example B3 | (B) 100 | (B) 95 | 7 | B | 18 | 47 | 100 | 2.61 | 5.56 |
| Example B4 | (B) 95 | (C) 90 | 7 | C | 20 | 55 | 100 | 2.75 | 5.00 |
| Example B5 | (B) 105 | (B) 100 | 7 | B | 16 | 45 | 100 | 2.81 | 6.25 |
| Example B6 | (B) 100 | (B) 95 | 7 | B | 20 | 60 | 100 | 3.00 | 5.00 |
| Example B7 | (B) 100 | (B) 95 | 7 | B | 16 | 50 | 100 | 3.13 | 6.25 |
| Example B8 | (C) 85 | (C) 85 | 7 | A | 3 | 10 | 77 | 3.33 | 25.67 |
| Example B9 | (C) 85 | (C) 85 | 7 | A | 4 | 9 | 79 | 2.25 | 19.75 |
| Example B10 | (C) 85 | (C) 85 | 7 | A | 3 | 11 | 78 | 3.67 | 26.00 |
| Example F11 | (A) 110 | (B) 105 | 7 | A | 10 | 25 | 84 | 2.50 | 8.40 |
| Example F12 | (A) 110 | (B) 105 | 7 | A | 9 | 23 | 82 | 2.56 | 9.11 |
| Example B13 | (C) 85 | (C) 75 | 7 | B | 16 | 38 | 82 | 2.38 | 5.13 |
| Example B14 | (C) 85 | (C) 75 | 7 | B | 17 | 35 | 85 | 2.06 | 5.00 |
| Example B15 | (C) 85 | (C) 75 | 7 | B | 18 | 37 | 88 | 2.06 | 4.89 |
| Example B16 | (B) 95 | (B) 95 | 7 | C | 22 | 51 | 100 | 2.32 | 4.55 |
| Example B17 | (B) 100 | (B) 95 | 7 | C | 20 | 48 | 100 | 2.40 | 5.00 |
| Example B18 | (C) 85 | (C) 85 | 7 | C | 28 | 60 | 100 | 2.14 | 3.57 |
| Example B19 | (B) 105 | (B) 105 | 7 | B | 24 | 58 | 100 | 2.42 | 4.17 |
| Example B20 | (B) 95 | (B) 90 | 7 | C | 25 | 54 | 100 | 2.16 | 4.00 |
| Example B21 | (B) 95 | (B) 90 | 7 | C | 28 | 53 | 100 | 1.89 | 3.57 |
| Example B22 | (B) 100 | (B) 95 | 7 | B | 16 | 38 | 98 | 2.38 | 6.13 |
| Example B23 | (B) 100 | (B) 95 | 7 | A | 11 | 35 | 95 | 3.18 | 8.64 |
| Example B24 | (B) 100 | (B) 95 | 7 | A | 12 | 33 | 92 | 2.75 | 7.67 |
| Example B25 | (B) 100 | (B) 95 | 7 | A | 10 | 34 | 90 | 3.40 | 9.00 |
| Example B26 | (B) 100 | (B) 95 | 7 | B | 17 | 39 | 97 | 2.29 | 5.71 |
| Example B27 | (B) 100 | (B) 95 | 7 | B | 18 | 47 | 100 | 2.61 | 5.56 |
| Example B28 | (B) 100 | (B) 95 | 7 | C | 20 | 50 | 100 | 2.50 | 5.00 |
| Example B29 | (B) 95 | (B) 90 | 7 | B | 10 | 23 | 96 | 2.30 | 9.60 |
| Comparative Example B1 | (D) 65 | (D) 65 | 7 | D | 75 | 100 | 100 | 1.33 | 1.33 |
| Comparative Example B2 | (D) 65 | (D) 65 | 7 | D | 88 | 100 | 100 | 1.14 | 1.14 |

As seen from the above results, the biodegradable resin particles of Examples have a biodegradation rate over

time, and a slower initial biodegradation rate as compared with the biodegradable resin particles of Comparative Examples.

In addition, it can be seen that the biodegradable resin particles of Examples have a higher oil absorption rate than that of the biodegradable resin particles of Comparative Examples.

Further, it can be seen that in the biodegradable resin particles of Examples, the biodegradable resin particles obtained by sequentially coating the base particles containing a specific plasticizer in a cellulose resin as a biodegradable resin with the first layer containing a cationic resin and the second layer containing a hydrophobic compound have biodegradability and are excellent in hydrolysis resistance.

The foregoing description of the exemplary embodiments of the present invention has been provided for the purposes of illustration and description. It is not intended to be exhaustive or to limit the invention to the precise forms disclosed. Obviously, many modifications and variations will be apparent to practitioners skilled in the art. The embodiments are chosen and described in order to best explain the principles of the invention and its practical applications, thereby enabling others skilled in the art to understand the invention for various embodiments and with the various modifications as are suited to the particular use contemplated. It is intended that the scope of the invention be defined by the following claims and their equivalents.

## Claims

**1.** A biodegradable resin particle, comprising:

a base particle containing a biodegradable resin which is a resin having an aerobic condition biodegradation rate of 50% or more after one month as measured by a method according to ISO-14855-2 (2018);
a first layer on a surface of the base particle, the first layer containing at least one cationic resin of a polyalkyleneimine, a polyallylamine and a polyvinylamine; and
a second layer on the first layer, the second layer containing an anionic or nonionic hydrophobic compound, wherein hydrophobic compound refers to a compound that imparts a water contact angle of a pellet in which the biodegradable resin particle is pelletized of 70° or more and 120° or less.

**2.** The biodegradable resin particle according to claim 1,
wherein the anionic or nonionic hydrophobic compound is at least one selected from the group consisting of a silicone compound, a hydrocarbon compound, a fatty acid compound, an acrylic resin, a polyester resin, and a urethane resin.

**3.** The biodegradable resin particle according to claim 2,
wherein the silicone compound is at least one selected from the group consisting of a dimethylpolysiloxane, a methylpolysiloxane, an MQ resin, and a silicone rubber.

**4.** The biodegradable resin particle according to claim 2 or 3,
wherein the hydrocarbon compound is at least one selected from the group consisting of a paraffin wax, a microcrystalline wax, a polyethylene wax, and a polypropylene wax.

**5.** The biodegradable resin particle according to any one of claims 2 to 4,
wherein the fatty acid compound is at least one selected from the group consisting of a carnauba wax, a rice wax, a candelilla wax, a palm wax, a castor oil wax, a soybean oil wax, and a sunflower oil wax.

**6.** The biodegradable resin particle according to any one of claims 1 to 5,
wherein the polyalkyleneimine is a polyalkyleneimine having a constituent unit having an alkylene group having 1 or more and 4 or less carbon atoms.

**7.** The biodegradable resin particle according to claim 6,
wherein the polyalkyleneimine having a constituent unit having an alkylene group having 1 or more and 4 or less carbon atoms is a polyethyleneimine.

**8.** The biodegradable resin particle according to any one of claims 1 to 7,
wherein the biodegradable resin is at least one selected from the group consisting of a cellulose resin and a polyester resin.

**9.** The biodegradable resin particle according to claim 8, wherein the biodegradable resin is the cellulose resin.

**10.** The biodegradable resin particle according to claim 9, wherein the cellulose resin is a cellulose acylate having two or more kinds of acyl groups.

**11.** The biodegradable resin particle according to claim 9 or 10,

wherein the base particle further contains at least one of a fatty acid ester and an aromatic compound, and the aromatic compound does not have a functional group that reacts with the cellulose resin and the aromatic compound has a long-chain aliphatic group and at least one of a phenolic hydroxy group and a monoglycidyl ether group that is directly bonded to an aromatic group of the aromatic compound.

**12.** The biodegradable resin particle according to claim 11, wherein the aromatic compound is a cardanol compound.

**13.** The biodegradable resin particle according to claim 12,

wherein the cardanol compound is at least one compound selected from the group consisting of a compound represented by the following general formula (CDN1) and a compound represented by the following general formula (CDN1-e),

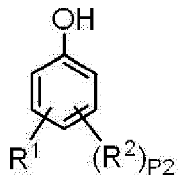

general formula (CDN1)

wherein, in the general formula (CDN1), $R^1$ represents an alkyl group which may have a substituent or an unsaturated aliphatic group which has a double bond and may have a substituent, $R^2$ represents a hydroxy group, a carboxy group, an alkyl group which may have a substituent, or an unsaturated aliphatic group which has a double bond and may have a substituent, P2 represents an integer of 0 or more and 4 or less, and a plurality of $R^2{}_S$ may be the same group or different groups when P2 is 2 or more,

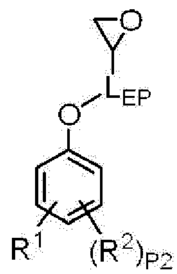

general formula (CDN1-e)

wherein, in the general formula (CDN1-e), $L^{EP}$ represents a single bond or a divalent linking group, $R^1$ represents an alkyl group which may have a substituent or an unsaturated aliphatic group which has a double bond and may have a substituent, $R^2$ represents a hydroxy group, a carboxy group, an alkyl group which may have a substituent, or an unsaturated aliphatic group which has a double bond and may have a substituent, P2 represents an integer of 0 or more and 4 or less, and a plurality of $R^2{}_S$ may be the same group or different groups when P2 is 2 or more.

**14.** The biodegradable resin particle according to any one of claims 11 to 13, wherein the cellulose resin is a cellulose acetate propionate and the aromatic compound is a cardanol compound.

**15.** The biodegradable resin particle according to any one of claims 11 to 13, wherein the cellulose resin is a cellulose acetate butyrate and the base particle contains the fatty acid ester.

**16.** The biodegradable resin particle according to any one of claims 1 to 15, wherein a mass ratio of a content of the cationic resin in the first layer to a content of the anionic or nonionic hydrophobic compound in the second layer is 0.1 or more and 10 or less.

**17.** The biodegradable resin particle according to claim 16,
wherein a content of the cationic resin with respect to the base particle is 0.1 mass% or more and 10 mass% or less.

**18.** The biodegradable resin particle according to any one of claims 1 to 17,
wherein a number average molecular weight of the cationic resin, determined by gel permeation chromatography, using GPC apparatus: HLC-8320GPC manufactured by Tosoh Corporation, column: TSKgelα-M, in terms of polystyrene using tetrahydrofuran, is 10,000 or more and 85,000 or less.

**Patentansprüche**

**1.** Biologisch abbaubares Harzteilchen, umfassend:

ein Basisteilchen, das ein biologisch abbaubares Harz enthält, das ein Harz mit einer biologischen Abbaurate unter aeroben Bedingungen von 50 % oder mehr nach einem Monat ist, gemessen nach einem Verfahren gemäß ISO-14855-2 (2018);
eine erste Schicht auf einer Oberfläche des Basisteilchens, wobei die erste Schicht mindestens ein kationisches Harz aus einem Polyalkylenimin, einem Polyallylamin und einem Polyvinylamin enthält; und
eine zweite Schicht auf der ersten Schicht, wobei die zweite Schicht eine anionische oder nichtionische hydrophobe Verbindung enthält, wobei sich hydrophobe Verbindung auf eine Verbindung bezieht, die einem Pellet, in dem das biologisch abbaubare Harzteilchen pelletisiert ist, einen Wasserkontaktwinkel von 70° oder mehr und 120° oder weniger verleiht.

**2.** Biologisch abbaubares Harzteilchen nach Anspruch 1,
wobei die anionische oder nichtionische hydrophobe Verbindung mindestens eine ist, die aus der Gruppe bestehend aus einer Silikonverbindung, einer Kohlenwasserstoffverbindung, einer Fettsäureverbindung, einem Acrylharz, einem Polyesterharz und einem Urethanharz ausgewählt ist.

**3.** Biologisch abbaubares Harzteilchen nach Anspruch 2,
wobei die Silikonverbindung mindestens eine ist, die aus der Gruppe bestehend aus einem Dimethylpolysiloxan, einem Methylpolysiloxan, einem MQ-Harz und einem Silikonkautschuk ausgewählt ist.

**4.** Biologisch abbaubares Harzteilchen nach Anspruch 2 oder 3, wobei die Kohlenwasserstoffverbindung mindestens eine ist, die aus der Gruppe bestehend aus einem Paraffinwachs, einem mikrokristallinen Wachs, einem Polyethylenwachs und einem Polypropylenwachs ausgewählt ist.

**5.** Biologisch abbaubares Harzteilchen nach einem der Ansprüche 2 bis 4,
wobei die Fettsäureverbindung mindestens eine ist, die aus der Gruppe bestehend aus einem Carnaubawachs, einem Reiswachs, einem Candelillawachs, einem Palmwachs, einem Rizinusölwachs, einem Sojabohnenölwachs und einem Sonnenblumenölwachs ausgewählt ist.

**6.** Biologisch abbaubares Harzteilchen nach einem der Ansprüche 1 bis 5,
wobei das Polyalkylenimin ein Polyalkylenimin mit einer konstituierenden Einheit mit einer Alkylengruppe mit 1 oder mehr und 4 oder weniger Kohlenstoffatomen ist.

**7.** Biologisch abbaubares Harzteilchen nach Anspruch 6,
wobei das Polyalkylenimin mit einer konstituierenden Einheit mit einer Alkylengruppe mit 1 oder mehr und 4 oder weniger Kohlenstoffatomen ein Polyethylenimin ist.

**8.** Biologisch abbaubares Harzteilchen nach einem der Ansprüche 1 bis 7,
wobei das biologisch abbaubare Harz mindestens eines ist, das aus der Gruppe bestehend aus einem Celluloseharz und einem Polyesterharz ausgewählt ist.

**9.** Biologisch abbaubares Harzteilchen nach Anspruch 8,

wobei das biologisch abbaubare Harz das Zelluloseharz ist.

**10.** Biologisch abbaubares Harzteilchen nach Anspruch 9,
wobei das Celluloseharz ein Celluloseacylat mit zwei oder mehr Arten von Acylgruppen ist.

**11.** Biologisch abbaubares Harzteilchen nach Anspruch 9 oder 10, wobei das Basisteilchen ferner mindestens eines von einem Fettsäureester und einer aromatischen Verbindung enthält, und die aromatische Verbindung keine funktionelle Gruppe aufweist, die mit dem Celluloseharz reagiert, und die aromatische Verbindung eine langkettige aliphatische Gruppe und mindestens eine von einer phenolischen Hydroxygruppe und einer Monoglycidylethergruppe, die direkt an eine aromatische Gruppe der aromatischen Verbindung gebunden ist, aufweist.

**12.** Biologisch abbaubares Harzteilchen nach Anspruch 11,
wobei die aromatische Verbindung eine Cardanolverbindung ist.

**13.** Biologisch abbaubares Harzteilchen nach Anspruch 12,

wobei die Cardanolverbindung mindestens eine Verbindung ist, die aus der Gruppe bestehend aus einer Verbindung, dargestellt durch die folgende allgemeine Formel (CDN1) und einer Verbindung, dargestellt durch die folgende allgemeine Formel (CDN1-e), ausgewählt ist,

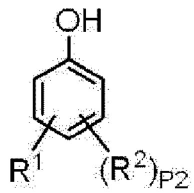
allgemeine Formel (CDN1)

wobei in der allgemeinen Formel (CDN1) $R^1$ eine Alkylgruppe, die einen Substituenten aufweisen kann, oder eine ungesättigte aliphatische Gruppe, die eine Doppelbindung aufweist und einen Substituenten aufweisen kann, $R^2$ eine Hydroxygruppe, eine Carboxygruppe, eine Alkylgruppe, die einen Substituenten aufweisen kann, oder eine ungesättigte aliphatische Gruppe, die eine Doppelbindung aufweist und einen Substituenten aufweisen kann, darstellt, P2 eine ganze Zahl von 0 oder mehr und 4 oder weniger darstellt, und eine Vielzahl von $R^2$ die gleiche Gruppe oder verschiedene Gruppen sein können, wenn P2 2 oder mehr ist,

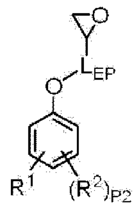
allgemeine Formel (CDN1-e)

wobei in der allgemeinen Formel (CDN1-e) $L^{EP}$ eine Einfachbindung oder eine zweiwertige Verknüpfungsgruppe darstellt, $R^1$ eine Alkylgruppe darstellt, oder eine ungesättigte aliphatische Gruppe, die eine Doppelbindung aufweist und einen Substituenten aufweisen kann, $R^2$ eine Hydroxygruppe, eine Carboxygruppe, eine Alkylgruppe, die einen Substituenten aufweisen kann, oder eine ungesättigte aliphatische Gruppe, die eine Doppelbindung aufweist und einen Substituenten aufweisen kann, darstellt, P2 eine ganze Zahl von 0 oder mehr und 4 oder weniger darstellt, und eine Vielzahl von $R^2$ die gleiche Gruppe oder verschiedene Gruppen sein können, wenn P2 2 oder mehr ist.

**14.** Biologisch abbaubares Harzteilchen nach einem der Ansprüche 11 bis 13,
wobei das Celluloseharz ein Celluloseacetatpropionat ist und die aromatische Verbindung eine Cardanolverbindung ist.

**15.** Biologisch abbaubares Harzteilchen nach einem der Ansprüche 11 bis 13,
wobei das Celluloseharz ein Celluloseacetatbutyrat ist und das Basisteilchen den Fettsäureester enthält.

**16.** Biologisch abbaubares Harzteilchen nach einem der Ansprüche 1 bis 15,
wobei ein Massenverhältnis eines Gehalts des kationischen Harzes in der ersten Schicht zu einem Gehalt der anionischen oder nicht ionischen hydrophoben Verbindung in der zweiten Schicht 0,1 oder mehr und 10 oder weniger beträgt.

**17.** Biologisch abbaubares Harzteilchen nach Anspruch 16,
wobei ein Gehalt des kationischen Harzes in Bezug auf das Basisteilchen 0,1 Masse-% oder mehr und 10 Masse-% oder weniger beträgt.

**18.** Biologisch abbaubares Harzteilchen nach einem der Ansprüche 1 bis 17,
wobei ein Zahlenmittel des Molekulargewichts des kationischen Harzes, bestimmt durch Gelpermeationschromatographie unter Verwendung einer GPC-Vorrichtung: HLC-8320GPC, hergestellt von Tosoh Corporation, Säule: TSKgel$\alpha$-M, bezogen auf Polystyrol unter Verwendung von Tetrahydrofuran, 10.000 oder mehr und 85.000 oder weniger beträgt.

## Revendications

**1.** Particule de résine biodégradable, comprenant :

une particule de base contenant une résine biodégradable qui est une résine ayant un taux de biodégradation en condition aérobie de 50 % ou plus après un mois, tel que mesuré par un procédé selon la norme ISO-14855-2 (2018) ;
une première couche sur une surface de la particule de base, la première couche contenant au moins une résine cationique d'une polyalkylèneimine, d'une polyallylamine et d'une polyvinylamine ; et
une seconde couche sur la première couche, la seconde couche contenant un composé hydrophobe anionique ou non ionique, dans laquelle le composé hydrophobe fait référence à un composé qui confère un angle de contact avec l'eau d'une pastille dans laquelle la particule de résine biodégradable est pastillée de 70° ou plus et 120 ° ou moins.

**2.** Particule de résine biodégradable selon la revendication 1, dans laquelle le composé hydrophobe anionique ou non ionique est au moins l'un choisi dans le groupe constitué d'un composé de silicone, d'un composé d'hydrocarbure, d'un composé d'acide gras, d'une résine acrylique, d'une résine de polyester et d'une résine d'uréthane.

**3.** Particule de résine biodégradable selon la revendication 2, dans laquelle le composé de silicone est au moins l'un choisi dans le groupe constitué d'un diméthylpolysiloxane, d'un méthylpolysiloxane, d'une résine MQ et d'un caoutchouc de silicone.

**4.** Particule de résine biodégradable selon la revendication 2 ou 3,
dans laquelle le composé hydrocarboné est au moins l'une choisie dans le groupe constitué d'une cire de paraffine, d'une cire microcristalline, d'une cire de polyéthylène et d'une cire de polypropylène.

**5.** Particule de résine biodégradable selon l'une quelconque des revendications 2 à 4,
dans laquelle le composé d'acide gras est au moins l'une choisie dans le groupe constitué d'une cire de carnauba, d'une cire de riz, d'une cire de candelilla, d'une cire de palme, d'une cire d'huile de ricin, d'une cire d'huile de soja et d'une cire d'huile de tournesol.

**6.** Particule de résine biodégradable selon l'une quelconque des revendications 1 à 5,
dans laquelle la polyalkylèneimine est une polyalkylèneimine ayant un motif constitutif ayant un groupe alkylène ayant 1 ou plus et 4 ou moins atomes de carbone.

**7.** Particule de résine biodégradable selon la revendication 6, dans laquelle la polyalkylèneimine ayant un motif constitutif ayant un groupe alkylène ayant 1 ou plus et 4 ou moins atomes de carbone est une polyéthylèneimine.

**8.** Particule de résine biodégradable selon l'une quelconque des revendications 1 à 7,
dans laquelle la résine biodégradable est au moins l'une choisie dans le groupe constitué d'une résine de cellulose et d'une résine de polyester.

**9.** Particule de résine biodégradable selon la revendication 8, dans laquelle la résine biodégradable est la résine de cellulose.

**10.** Particule de résine biodégradable selon la revendication 9, dans laquelle la résine de cellulose est un acylate de cellulose ayant deux types de groupes acyle ou plus.

**11.** Particule de résine biodégradable selon la revendication 9 ou 10,

dans laquelle la particule de base contient en outre au moins l'un d'un ester d'acide gras et d'un composé aromatique, et
le composé aromatique n'a pas de groupe fonctionnel qui réagit avec la résine de cellulose et le composé aromatique a un groupe aliphatique à longue chaîne et au moins l'un d'un groupe hydroxy phénolique et d'un groupe monoglycidyléther qui est directement lié à un groupe aromatique du composé aromatique.

**12.** Particule de résine biodégradable selon la revendication 11, dans laquelle le composé aromatique est un composé de cardanol.

**13.** Particule de résine biodégradable selon la revendication 12,

Dans laquelle le composé de cardanol est au moins un composé choisi dans le groupe constitué d'un composé représenté par la formule générale suivante (CDN1) et d'un composé représenté par la formule générale suivante (CDN1-e),

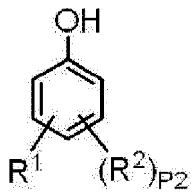 formule générale (CDN1)

dans laquelle, dans la formule générale (CDN1), $R^1$ représente un groupe alkyle qui peut avoir un substituant ou un groupe aliphatique insaturé qui a une double liaison et peut avoir un substituant, $R^2$ représente un groupe hydroxy, un groupe carboxy, un groupe alkyle qui peut avoir un substituant, ou un groupe aliphatique insaturé qui a une double liaison et peut avoir un substituant, P2 représente un nombre entier de 0 ou plus et 4 ou moins, et une pluralité de $R^2$ peuvent être le même groupe ou différents groupes lorsque P2 vaut 2 ou plus,

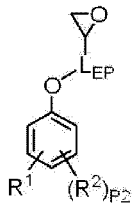 formule générale (CDN1-e)

dans laquelle, dans la formule générale (CDN1-e), $L^{EP}$ représente une liaison simple ou un groupe de liaison divalent, $R^1$ représente un groupe alkyle qui peut avoir un substituant ou un groupe aliphatique insaturé qui a une double liaison et peut avoir un substituant, $R^2$ représente un groupe hydroxy, un groupe carboxy, un groupe alkyle qui peut avoir un substituant, ou un groupe aliphatique insaturé qui a une double liaison et peut avoir un substituant, P2 représente un nombre entier de 0 ou plus et 4 ou moins, et une pluralité de $R^2$ peuvent être le même groupe ou différents groupes lorsque P2 vaut 2 ou plus,

**14.** Particule de résine biodégradable selon l'une quelconque des revendications 11 à 13, dans laquelle la résine de cellulose est un acétate propionate de cellulose et le composé aromatique est un composé de cardanol.

**15.** Particule de résine biodégradable selon l'une quelconque des revendications 11 à 13,

dans laquelle la résine cellulosique est un acétate butyrate de cellulose et la particule de base contient l'ester d'acide gras.

**16.** Particule de résine biodégradable selon l'une quelconque des revendications 1 à 15, dans laquelle un rapport massique d'une teneur en résine cationique de la première couche à une teneur en composé hydrophobe anionique ou non ionique de la seconde couche est de 0,1 ou plus et de 10 ou moins.

**17.** Particule de résine biodégradable selon la revendication 16, dans laquelle une teneur en résine cationique par rapport à la particule de base est de 0,1 % en masse ou plus et de 10 % en masse ou moins.

**18.** Particule de résine biodégradable selon l'une quelconque des revendications 1 à 17, dans laquelle un poids moléculaire moyen en nombre de la résine cationique, déterminé par chromatographie par perméation de gel, à l'aide d'un appareil GPC : HLC-8320GPC fabriqué par Tosoh Corporation, colonne : TSKgelα-M, en termes de polystyrène utilisant du tétrahydrofurane, est de 10 000 ou plus et de 85 000 ou moins.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004256579 A
- JP 2015512863 T
- JP 2011225643 A
- WO 2013166385 A1